# EUROPEAN PATENT APPLICATION

(11) **EP 2 062 944 A1**
(43) Date of publication of application: **27.05.2009**
(21) Application number: 07022521.4
(22) Date of filing: 20.11.2007
(51) Int. Cl.: C09B 5/62, G01N 33/533, G01N 33/58

(54) **Water-soluble rylene dyes, methods for preparing the same and uses thereof as fluorescent labels for biomolecules**

(71) Applicant: Max-Planck-Gesellschaft zur Förderung der Wissenschaften e.V., 80539 München (DE)
(72) Inventor: Peneva, Kalina, 55122 Mainz (DE); Herrmann, Andreas, Dr., 65199 Mainz (DE); Müllen, Klaus, Prof. Dr., 50939 Köln (DE)
(74) Representative: Katzameyer, Michael

(57) **Abstract**

The present invention relates to novel rylene diimide compounds, a method for preparing said compounds, the use of said compounds as fluorescent label for biomolecules as well as a method for purifying bioconjugates.

## Description

Fluorescence microscopy is a widely used and effective tool for visualizing subcellular structures and for localizing proteins within cells. Fluorescence is ideally suited for observing the location of molecules and cells as it is non-invasive and can be detected with high sensitivity and signal specificity. The spectroscopic properties of fluorescence can be exploited to obtain information not only on the location of labeled macromolecules but also on the immediate molecular environment.

Single-molecule spectroscopy has gone beyond that and has revealed information about complex biological molecules and processes which are difficult to obtain from ensemble measurements. Single proteins, variants, drugs or other single bioparticles were labelled and their pathway and interactions were followed inside living cells (e.g., G. Seisenberger, et al., Science 2001, 294, 1929).

For observing biological entities on the single molecule level the properties of the label are crucial. It should be water-soluble, highly fluorescent in aqueous environment, exhibiting high photochemical and thermal stability, enable scalable production and should have a reactive group for coupling to the biomolecule, e.g. protein or enzyme. Moreover, the attachment of the coupling group should not affect the biomolecule's structure or function, or in the case of an enzyme, its activity. In particular, an exceptional photostability of the label is needed to visualize or track the biomolecule for a sufficient period of time.

Rylene chromophores have proven to be a remarkable class of dyes characterized by an exceptional thermal and photochemical stability as well as fluorescence quantum yields close to unity in organic solvents (A. Dubois et al., Appl. Opt. 1996, 35, 3193; 1, 303) and several members of the rylene family have been described and tested in single molecule molecule experiments (e.g. H. Uji-i et al., Polymer 2006, 47, 2511).

In Jung et al. (J. Am. Chem. Soc. 2006, 128, 5283-5291) terrylene diimide dyes are disclosed which are functionalized with a monocarboxylic acid for applications in single molecule studies and membrane labelling. However, the disadvantage of these dyes is their low coupling efficiency to biomolecules and, thus, their low yield of labelling of said biomolecules. Further, in order to covalently attach the monocarboxylic acid-functionalized terrylene diimide dye to biomolecules, the use of an additional coupling reagent such as 1-ethyl-3-[3-dimethylaminopropyl]carbodiimide (EDC) is required. Due to their very high reactivity, these coupling reagents very often lead to the formation of undesired side products which are often difficult to remove, thus complicating the protein labelling procedure.

DE 103 08 941 describes monofunctionalized perylene tetracarboxylic acid diimides (PDI), wherein the linking group, which carries the coupling group, is bound to the nitrogen atom of the imide group of the perylene via a phenyl group. However, due to the phenyl group, the perylene tetracarboxylic acid diimide has a decreased reactivity with the biomolecule to be labeled.

It should be also noted that PDI dyes have a strong tendency to form aggregates in aqueous solution (G. Schnurpfeil, J. Stark, D. Wöhrie, Dyes Pigm. 1995, 720, 339-350; H. Langhals, German Patent DE-37 035 13, 1987) and the combination of good water solubility and high fluorescence quantum yields still represents a challenging goal.

Therefore, a main object of the present invention is to provide novel water-soluble rylene diimide dyes with improved properties regarding reactivity with biomolecules such as higher reactivity and/or site-specifity, coupling efficiency and thus labelling yield of the biomolecules to be labeled, and outstanding photostability of the dyes associated with extended survival times under strong illumination conditions. Such dyes would i.a. allow a tracking of single interfacial enzymes, such as phospholipases, with their complex catalytic pathways and modes of operation on their native substrate which has not been performed yet in the prior art.

A related, more specific object is to provide novel rylene dyes suitable for the site-specific labelling of proteins. Prior to the present invention, such rylene dyes have not been described in the literature.

A further object is to provide improved synthetic strategies for preparing the novel rylene dyes. A still further object is to provide the use of these dyes as improved labels for biomolecules and to provide a new and advantageous purification method for bioconjugates comprising such labels.

These objects have been achieved in accordance with the present invention by providing the rylene diimide compounds according to claims 1 and 12, the methods of preparation acording to claims 13, 17 and 18, the conjugates of claim 19, the uses of claims 21, 22, and the purification method of claim 25. Specific and/preferred embodiments are the subject of further claims.

Formula (I) as defined in claim 1: 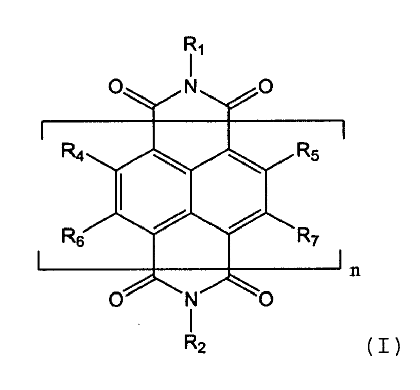 wherein R₁ is an organic residue which is attached to the nitrogen atom via a carbon atom,
n is an integer from 1 to 6, preferably from 2 to 4,
R₂ is -(CH₂)ₘ-A-(CH₂)ₒ-B,
m is an integer from 0 to 20, preferably from 1 to 10, e.g.
0, 1 to 3, 1 to 5, 5 to 10, 11 to 20,
o is an integer from 0 to 20, preferably from 0 to 10, e.g.
0, 1 to 3, 1 to 5, 1 to 10, 5 to 10, 11 to 20,
A is a linking group,
B is a coupling group selected from the group consisting of isothiocyanate, activated ester, carbonyl azide, sulfonyl chloride, aldehyde, alkyl halide, haloacetamide, maleimide, nitrilotriacetic acid, disulfide, thiosulfate, thioester, and thiol,
and R₄, R₅, R₆ and R₇ are independently hydrogen or an organic residue, which carries at least one negatively or positively charged group in neutral medium or at least one hydrophilic uncharged group which is preferably selected from a hydroxyl group and polyoxy-C₂-C₄-alkyl groups, preferably polyethylene groups with 3 or more, e.g. up to 50 or 100 alkyloxide units, wherein at least two of R₄, R₅, R₆ and R₇ are an organic residue, which carries at least one negatively or positively charged group in neutral medium or at least one hydrophilic uncharged group which is preferably selected from a hydroxyl group and polyoxy-C₂-C₄-alkyl groups, preferably polyethylene groups with 3 or more, e.g. up to 50 or 100 alkyloxide units. More preferably, at least two of R₄, R₅, R₆ and R₇ are an organic residue which carries at least one negatively or positively charged group in neutral medium.

The advantage of R₂ being -(CH₂)ₘ-A-(CH₂)ₒ-B lies in the fact that R₂ has a high reactivity with biomolecules. In particular, R₂ does not contain any phenyl group which might decrease the reactivity of the rylene diimide compounds.

Thhe rylene diimide compounds of formula (I) of the present invention having the coupling groups B as defined above, consequently show a high coupling efficiency to biomolecules and, thus, a high labelling yield. Another advantage of the rylene diimide compounds of formula (I) of the present invention is that by using the coupling groups defined as B no additional coupling reagent is necessary in order to covalently attach the dye to the biomolecules to be labeled. This is advantageous in respect to time and costs of the labelling procedure of the biomolecules.

In one preferred embodiment, the coupling group is an activated ester, in particular an N-hydroxysuccinimide ester, or a maleimide. For these coupling groups, a very high coupling efficiency, high absorption and fluorescence maxima as well as fluorescence quantum yields could be achieved; e.g. a fluorescence quantum yield of - 0.40 for compound 3 and 0.58 for 5 (cf. Table 1 in Example 1). The maleimide coupling group may also be used for site-specific labelling of proteins. The maleimide-functionalized chromophore can be covalently attached to the thiol residue from any cysteine of the protein via Michael adddition. A site-specific labelling is obtained if the protein does not comprise more than one cysteine residue in the molecule. If no cysteine residue is present in the native protein, it may be possible to introduce a non-native cysteine at a desired location.

A further advantage of an N-hydroxysuccinimide activated ester group is that the leaving group is non-toxic. This is an important fact with respect to the use of the dyes for in vivo labelling. Requirements for *in vivo* labelling such as non-toxicity limit the use of activated ester group functions for the labelling of biomolecules. Further, the N-hydroxysuccinimide activated ester group provides for a high labelling efficiency. A further advantage of the reactive functionalities B used in the present invention is that the dyes can be attached to a variety of biomolecules, in particular proteins. Examples of precursors for preparing the reactive functionalities are carboxylic acid groups or amino groups which can be converted into reactive functionalities by methods known in the art.

In another preferred embodiment, the coupling group is a thioester or thiol. The thioester group reacts specific with N-terminal cysteine residues and thus allows a site specific-labelling of proteins, e.g. enzymes, at the N-terminus. The introduction of fluorescent reporters at specific sites of a target molecule is desirable or even necessary for a number of spectroscopic methods for investigating the structure and function of target molecules, in particular proteins such as enzymes. More specifically, it is a prerequisite for the application of fluorescence resonance energy transfer (FRET) techniques, an important tool for, e.g., studying conformational distributions and dynamics of biomacromolecules such as proteins. Prior to the present invention, no fluorescent rylene derivative was available for covalently labelling either the N- or the C-terminus of proteins selectively.

Preferably, the thioester coupling group is represented by the formula -COSR, wherein R is C₁-C₄-alkyl, C₆-C₁₀-aryl or C₇-C₁₅-aralkyl, which may be optionally substituted with sulphonate, or the group -(CH₂)₂-CONH₂. In one specific, but non-limiting example R is benzyl.

The thioester-functionalized rylene dyes of the present invention exhibit both an excellent water solubility and a high fluorecent quantum yield in aqueous medium (e.g. 54% for compound **9,** compare Example 4). Moreover, after binding of a thioester-functionalized rylene dye to a protein (Example 5), the brightness of the rylene dye decreased only slightly. This indicates an almost independent fluorescence quantum yield from the local protein environment, which is a rather rare and desirable property for labelling dyes and makes the these dyes a very attractive new label for N-terminal cysteines in proteins.

Preferred, but non-limiting examples for the thiol coupling group are a 1,2-aminothiol group and cysteine. The use of a thiol coupling group is based on the same ligation reaction between a thioester group and a thiol group as outlined above. However, in this case the functionalities of dye and protein are reversed and this embodiment requires that the protein has a thioester group introduced to provide a coupling partner for the thiol group.

In a further preferred embodiment, the coupling group is a nitrilotriacetic acid moiety. This group, in the presence of divalent metal ions such as Ni²⁺ etc., is capable to form stable complexes with ligands from specific protein functionalities such as His tags. Thus, a specific labelling of proteins at the site of a previously introduced polyhistidin tag is possible. This option has great practical relevance since polyhistidin tags are the most widely used genetically encoded tags. A polyhistidine tag usually consists of 2-6 consecutive histidine residues and was originally developed for the purification of recombinant proteins by immobilized metal-affinity chromatography. Different modifications of this molecular recognition technique have been exploited including target protein detection and protein structure studies. This strategy has been also utilized to introduce a chromophore containing a nitrilotriacetic acid (NTA) moiety into His-tagged proteins (Lata et al., J. Am. Soc. 2006, 128, 2365-2372; Kapanidis et al., J. Am. Soc. 2001, 123, 12123-12125). All of the reported chromophores, however, suffered from the same drawback - a severe loss of the fluorescence upon binding of the paramagnetic nickel ion. When Ni²⁺ was complexed with the NTA moiety that was attached to commercial Cy3 and Cy5 dyes, the fluorescence quantum yield dropped by 75% (Kapanidis et al.). Although the quenching of the fluorescence is distance dependant, a 80% loss of the fluorescence of the dye ATTO 565 was measured, even when a longer spacer between the NTA moiety and the fluorophore was introduced (Lata et al.).

With the NTA-modified rylene dyes of the present invention, it is possible to overcome the disadvantages of the prior art. In particular, in contrast to the above findings, Examples 6 and 7 of the present specification clearly demonstrate that the photophysical properties of the inventive NTA-modified dyes remain unchanged upon Ni²⁺ binding.

In a preferred embodiment of the present invention, R₁ being a component of the imide group is bound to the nitrogen atom of the imide via a secondary or tertiary C atom, whereas also a binding via a primary C atom is possible, i.e. via a CH₂ group, Particularly preferably, R₁ is a secondary or tertiary aliphatic residue or cyclic residue having 3 to 30 carbon atoms, in particular, mono- or bicyclic aromatic or heteroaromatic residues such as phenyl, pyridyl or naphthyl residues which optionally contain one or more substituents.

Examples of suitable substituents are CN, NO₂, halogen (e.g. F, Cl, Br or I), OH, OR⁹, OCOR⁹, SH, SR⁹, SCOR⁹, SO₂R⁹, CHO, COR⁹, COOH, COOM, COOR⁹, CONH₂, CONHR⁹, CON (R⁹)₂, SO₃H, SO₃M, SO₃R⁷, NH₂, NHR⁹ or N(R⁹)₂, wherein M is a cation, e.g. an alkali metal ion such as sodium, potassium, etc.; and R⁹ is an optionally halogen-substituted linear or branched chain C₁-C₆ alkyl moiety. Cyclic residues, e.g. aromatic or heteroaromatic residues, may additionally be substituted by one or more residues R⁹.

At least two, preferably at least three and more preferably at least four of residues R₄, R₅, R₆ and R₇ are an organic residue which carries a charged group or a hydrophilic uncharged group which is preferably selected from a hydroxyl group and polyoxy-C₂-C₄-alkyl groups, preferably polyethylene groups with 3 or more, e.g. up to 50 or 100 alkyloxide units. More preferably, at least two, preferably at least three and more preferably at least four of residues R₄, R₅, R₆ and R₇ are an organic residue which carries at least one negatively or positively charged group in neutral medium.

Preferably, the organic residue is an acyclic or cyclic residue of 1 to 30 carbon atoms, more preferably 6 to 20 carbon atoms,
wherein the carbon atoms are optionally substituted with heteroatoms, preferably 1 to 4 heteratoms selected from the group consisting of oxygen, nitrogen and sulfur. More preferably, the heteratom is nitrogen. Preferably, all residues R₄, R₅, R₆ and R₇, which are not hydrogen, carry a charged group. More preferably, the charged group is a hydrophilic group.

In one preferred embodiment, the charged group is in neutral medium, e.g. at pH 7, a positively charged group. Suitable, but non-limiting examples are amino or ammonium groups, such as a quaternary ammonium group or an alkylated heteroaromatic N atom, e.g. an N-alkyl-pyridinium, N-alkyl-quinolinium or N-alkyl-isoquinolinium group, wherein the alkyl residue preferably contains up to 6 carbon atoms and may be substituted as well.

In another preferred embodiment, the charged group is in neutral medium, e.g. at pH 7, a negatively charged group. Examples of suitable negatively charged groups are sulfonic acid and/or carboxylic acid groups, SO₃H and COOH as well as the salts SO₃M and COOM thereof, wherein M is a cation, e.g. an alkali metal ion such as potassium or sodium.

The positively charged groups or negatively charged groups, such as sulfonic acid and carboxylic acid groups, or hydrophilic uncharged groups are responsible for the water solubility of the rylene dyes. However, due to the large n-electron system of the rigid hydrophobic core, many of the rylene dye molecules are still stacked, forming water-soluble aggregates. The ambivalent nature of these molecules offers additional interesting properties, since the rylene core presents a strong affinity to, for example, lipid membranes. This property makes rylenes an exceptional label for artificial as well as biological membranes.

It is further preferred that at least two of R₄, R₅, R₆ and R₇ comprise aromatic residues, in particular, monocyclic or bicyclic residues such as phenyl.

In a particularly preferred embodiment, at least two of R₄, R₅, R₆ and R₇ are represented by the general formula (II)

-O-Ar (II),

wherein Ar is an aromatic residue which contains at least one charged group that is as defined above. Particularly, at least two of R₄, R₅, R₆ and R₇ have the general structural formula (III): 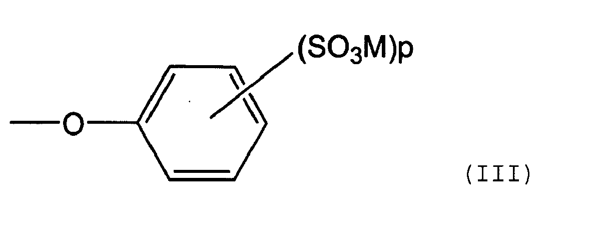 wherein M is a cation and p is an integer from 1 to 3.

The rylene diimide compounds of formula (I) comprise 1 to 6 units n, wherein n is defined as the structure of formula (I) in the brackets , i.e. n = 1 represents naphthylene, n = 2 represents perylene, n = 3 represents terrylene, n = 4 represents quaterrylene, n = 5 represents pentarylene, n = 6 represents hexarylene, and derivatives thereof, which may be substituted or unsubstituted. More specifically, the rylene compounds of formula (I) have 2 to 4 units n and, thus, comprise one of the following basic structures:

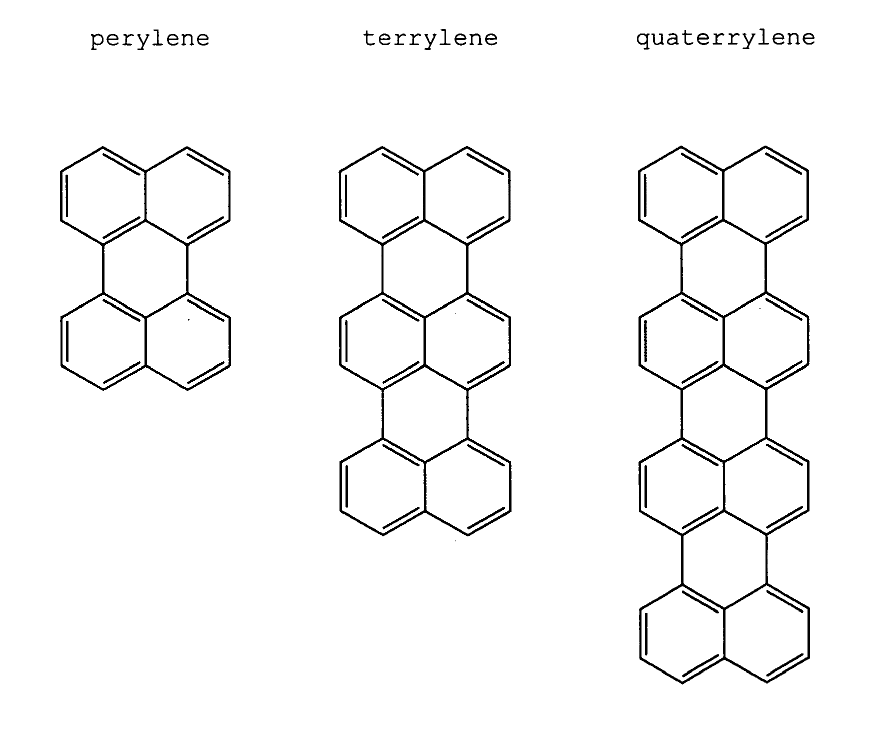

The above basic structures are more preferably substituted with residues R⁴, R⁵, R⁶ and R⁷ as follows, wherein R⁴, R⁵, R⁶ and R⁷ are most preferably not hydrogen:

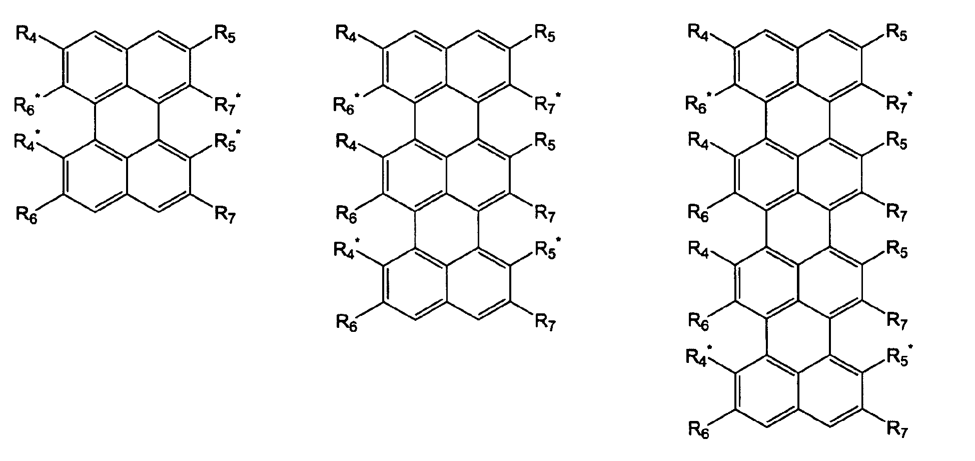

More preferably, the rylene diimide compound of formula (I) is a perylene or terrylene dye and, most preferably, a terrylene dye. Advantageously, the terrylene dyes absorb above the wavelength of 600 nm, whereas the absorption region of perylene dyes lies below 600 nm. The wavelength of above 600 nm is ideal for single molecule and live-ell imaging experiments because autofluorescence of cellular components at that wavelength region is minimal and a better-signal-to-noise ratio is obtained. In addition, it has become evident that terrylene is even more photostable than perylene. Thus, water-soluble terrylene derivatives of the invention have a particularly high survival time (offering the possibility of studying biological processes which require longer observation periods), a longer fluorescent time, a high photostability and are thus very advantageous for biological applications and ideal fluorophosphores for single molecule and live-cell imaging experiments.

In a preferred embodiment of the present invention, the linking group A is independently selected from 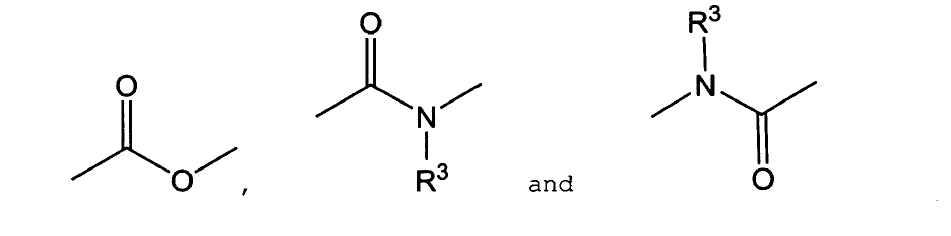 wherein R³ is independently selected from hydrogen, linear or branched-chain C₁-C₁₀ alkyl. Preferably, R³ is hydrogen.

Most preferably, the rylene diimide dyes of the present invention are selected from N-(2,6-diisopropylphenyl)-N'-(3-N-succinimidyl)carboxyethyl)-1,6,7,12-tetra(4-sulfophenoxy)-perylene-3,4:9,10-tetracarboxydiimide (compound **5** in Scheme 1 of Example 1), N-(2,6-diisopropylphenyl)-N'(5-(N-(2-(4-maleimidobutyryl)ethyl)-carboxypentyl)-1,6,9,14-tetra(4-sulfonylphenoxy)-terrylene-,3,4,11,12-tetracarboxydiimide (compound **8** in Scheme 1 of Example 1), N-(2,6-diisopropylphenyl)-N'-[N-{4-(benzylthio)-4-oxobutanoyl}aminoethyl]-1,6,7,12-tetra(4-sulfophenoxy)-perylene-3,4:9,10-tetracarboxydiimide (compound **9** in Scheme 2) or N-(2,6-diisopropylphenyl)-N'-[3-(N-(5-(amino-1-carboxypentyl)iminodiacetyl)carboxyethyl]-1,6,7,12-tetra(4-sulfophenoxy)-perylene-3,4:9,10-tetracarboxydiimide (compound **10** in Scheme 3).

A further aspect of the invention relates to a rylene diimide compound of formula (I) 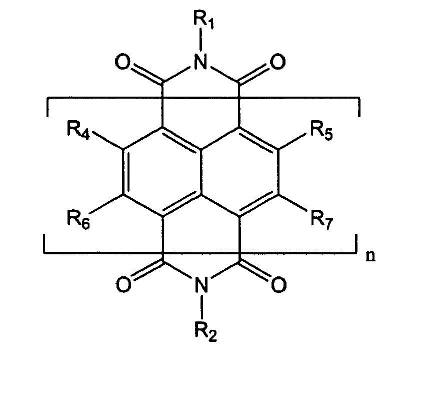 wherein R₁ and R₂ are independently an organic residue which is attached to the nitrogen atom via a carbon atom,
n is an integer from 3 to 4,
wherein at least one of R₁ and R₂ contains a coupling group B selected from the group consisting of isothiocyanate, activated ester, carbonyl azide, sulfonyl chloride, aldehyde, alkyl halide, haloacetamide, maleimide, nitrilotriacetic acid, disulfide, thiosulfate, thiol and thioester,
and R₄, R₅, R₆ and R₇ are independently hydrogen or an organic residue, which carries at least one negatively or positively charged group in neutral medium or at least one hydrophilic uncharged group which is preferably selected from a hydroxyl group and polyoxy-C₂-C₄-alkyl groups, preferably polyethylene groups with 3 or more, e.g. up to 50 or 100 alkyloxide units, wherein at least two of R₄, R₅, R₆ and R₇ are an organic residue, which carries at least one negatively or positively charged group in neutral medium or at least one hydrohilic uncharged group which is prferably selected from a hydroxyl group and polyoxy-C₂-C₄-alkyl groups, preferably polyethylene groups with 3 or more, e.g. up to 50 or 100 alkyloxide units. More preferably, R₄, R₅, R₆ and R₇ are independently at each occurence hydrogen or an organic residue, which carries at least one negatively or positively charged group in neutral medium, wherein at least two of R₄, R₅, R₆ and R₇ are an organic residue, which carries at least one negatively or positively charged group in neutral medium.

A further embodiment of the invention relates to a method for preparing a rylene diimide compound of formula (I), comprising the steps:
(a) optionally reacting a rylene diimide compound of formula (IV) 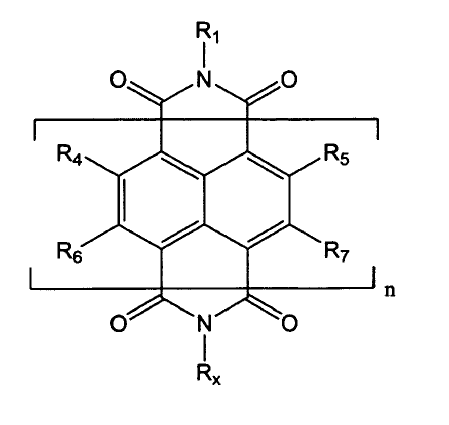 wherein R₁ and Rₓ are independently an organic residue which is attached to the nitrogen atom via a carbon atom and R₄, R₅, R₆ and R₇ are independently at each occurence hydrogen or an organic residue, to give a compound of formula (V), 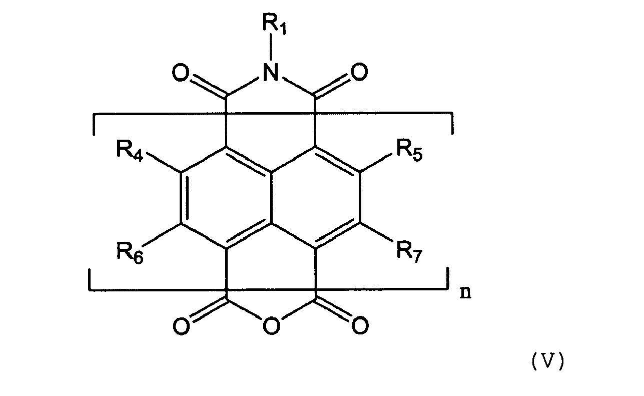
(b) reacting a compound of formula (V), to give a compound of formula (VI) 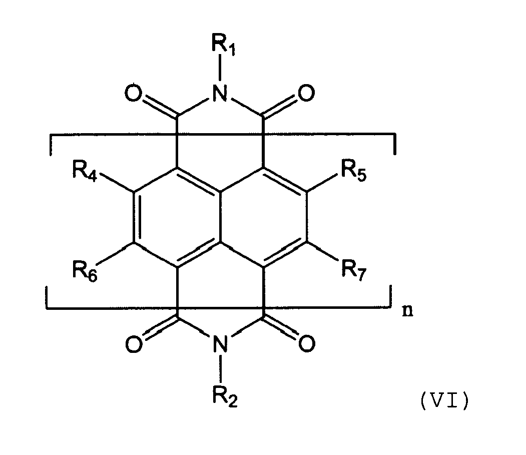 wherein R₂ is -(CH₂)_{q}-COOH or -(CH₂)_{q}-NHR⁸, wherein q is an integer from 0 to 20, preferably from 1 to 10, and R⁸ is selected from hydrogen, linear or branched-chain C₁-C₁₀ alkyl,
(c) reacting a compound of formula (VI), to give a rylene diimide compound of the above formula (I), wherein R₂ is -(CH₂)ₘ-A-(CH₂)ₒ-B, m is an integer from 0 to 20, preferably 1 to 10, e.g. 0, 1 to 3, 1 to 5 or 11 to 10, o is an integer from 0 to 20, preferably 0 to 10, e.g. 0, 1 to 3, 1 to 5 or 1 to 10, A is 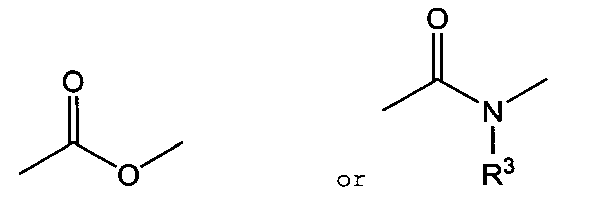 B is a coupling group selected from the group consisting of isothiocyanates, activated esters, carbonyl azides, sulfonyl chlorides, aldehydes, alkyl halides, haloacetamides, imides, nitriotriacetic acid, disulfides, thiosulfates, thiols and thioesters, and
(d) optionally further reacting the compound of formula (I) from step(b), wherein A is 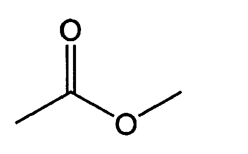 to give a compound of formula (I), wherein A is

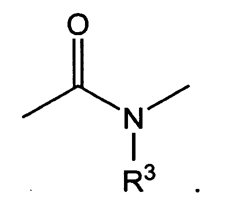

For the present perylene dyes, step a) preferably comprises the saponification of the Rₓ residue of formula (IV) under basic conditions, more preferably under strong basic conditions, and the removal of the imide group of Rₓ, to give perylene monoimide monoanhydride compounds of formula (V). Subsequently, in step b) the monoanhydride group of the compound of formula (V) is removed by an imidization reaction with 1,2-alkylene diamine, e.g. 1,2-ethylene diamine, or 3-aminoalkanoic acid, e.g. 3-aminopropanoic acid, respectively, to give monofunctionalized perylenes of formula (VI), wherein R₂ is -(CH₂)_{q}-COOH or -(CH₂)_{q}-NHR⁸, respectively. In step c), at least two, more preferably, all of the residues R₄, R₅, R₆ and R₇ of the compound of formula (VI) are then provided with at least one charged group, e.g. by sulfonating under acidic conditions. Also in step c), the compound of formula (VI) is further functionalized with a coupling group, to give a compound of formula (I).

In one preferred embodiment, the coupling group is an activated ester group and the final product (I) is obtained by reacting compound (VI) as defined above or derivative thereof (e.g. a sulfonated derivative thereof) wherein R₄, R₅, R₆ and R₇ are as defined for formula (I) in claim 1, with a compound which provides the activated ester moiety, such as 4-maleimidobutyric acid N-succinimidyl ester (GMBS) in the case of an amino-functionalized compound (VI) or N,N,N'N'-tetramethyl-(succinimido) uronium tetrafluoroborate (TSTU) in the case of an carboxy-functionalized compound (VI).

In the specific embodiment of the present invention wherein the coupling group is a thioester, a compound of formula (VI)
wherein R₄, R₅, R₆ and R₇ are as defined for formula (I) in claim 1 is reacted first with an activated thioester compound, e.g. thiolane-2,5-dione, and the resulting intermediate is reacted with an aryl or alkyl halide to give a compound of formula (I).

In an specific embodiment of the present invention wherein the coupling group is a nitrilotriacetic acid (NTA) group, the compound of formula (I), wherein B is an activated ester group, is further reacted in solution with a compound providing a nitrilotriacetic acid moiety, e.g. t-butyl-protected NTA. After removal of the protecting groups, the compound (I) wherein B is a NTA group is obtained (scheme 3a). Alternatively, this NTA-functionalized dye may be prepared by a solid-phase-approach as outlined in reaction scheme 3b of Example 6.

In the above preparation of the rylene, in particular perylene diimide compounds of the present invention, a high solubility of the intermediate compound of formula (VI) in the organic solvent such as dichloromethane or dichloromethane/acetone is of importance with respect to the further purification of the intermediate. This high solubility of the intermediate of formula (VI) is attained by the specific preparation process of the present invention, namely a first saponification step under strong basic conditions, wherein the imide group, which is attached to the residue Rₓ is removed, to give a monoanhydride group, and by reacting the monoanhydride group in an imidization reaction to the intermediate compound of formula (VI). In contrast, in the conventional preparation methods of rylene diimides, a rylene dianhydride compound is reacted first in a statistic imidization reaction, to give a rylene diimide compound, wherein the linking group which is attached to the nitrogen atom of the imide contains a phenyl group. However, this intermediate compound has a lower solubility in the organic solvent such as, e.g., toluene, and, thus, the degree of purification is lower (see DE 103 08 941).

Thus, there is a need in the art to provide a preparation process for rylene, in particular perylene, diimide compounds, wherein this problem is avoided and, in particular, wherein no intermediates are produced which have low solubility in the respective solvent.

This object is achieved in accordance with the present invention by the above preparation process, wherein, first, a saponification step is performed and, second, an imidization step, and wherein the intermediates in each step are better soluble in the organic solvents and thus the purification can be performed more easy and effective in each step. Also the overall yields of intermediates and final products are higher with the above process of the ivention.

The present terrylene dyes of formula (I) may be prepared analogous to the perylene dyes. Alternatively, the starting compound may be a terrylene diimide compound such as Compound 6 in Scheme 1b which is functionalized with a monocarboxylic acid. Compound 6 and similar compounds can be prepared analogous to the method disclosed in Jung et al. (J. Am. Chem. Soc. 2006, 128, 5283-5291). The carboxylic acid function may than be converted into an activated ester group or other reactive functionalities using methods known in the art. Optionally, an activated ester group may be further reacted in an amidization reaction to a carboxylic acid amide group.

In a further embodiment, the present invention relates to the use of the above-described compounds of formula (I) as fluorescent label for biomolecules, in particular, as fluorescent label for proteins. In a preferred embodiment, the biomolecules are enzymes.

The use of the inventive compounds as fluorescent label for biomolecules is preferably accomplished by coupling the monofunctionalized compounds of formula (I) carrying the variety of different reactive functionalities, defined as group B, covalently or, in the case of the NTA coupling group, non-covalently to a binding partner, e.g. a biomolecule. For example, an N-hydroxysuccinimide perylene derivative targeting lysins may be used for the labelling of the enzyme phospholipase, a thioester derivative may be used for the labelling of plasminogen activator inhibitor-1 and an NTA derivative may be used for the labelling of His₆ tagged ATP synthase. Thus, the present invention also relates to conjugates of compounds of formula (I) with a binding partner, in particular, a biomolecule such as a protein, in particular an enzyme.

The compounds of the present invention may also suitable for the use in other technical fields wherein rylene diimide compounds have been successfully enployed, e.g. in dye lasers, as labelling groups in analytical processes, as tracer in scintillation counters, in fluorescence solar collectors, in liquid crystals, in cold light sources, in material testing, as photoconductors, in photographic processes, in illumination and display elements, as semiconductors, etc.

The compounds or the conjugates thereof, e.g. covalent conjugates with biomolecules, such as enzymes, nucleic acids, proteins, peptides, saccharides, etc. can be dissolved in fluids, e.g. organic or/and aqueous solvents, or in solids, e.g. plastics.

In another aspect, the present invention is directed to a method for purifying bioconjugates. The conventional purification methods of bioconjugates rely on size exclusion and/or affinity chromatography. The separation principles are based on the size of the biomolecules and/or on intermolecular binding forces between the biomolecule to be separated and the ligand immobilized to an insoluble inert carrier. However, the procedure of size exclusion or affinity chromatography is inconvenient and time-consuming. Thus, there is a need in the art to provide a purification method for bioconjugates which is easy and fast to perform.

This object is achieved by a purification method for bioconjugates which contain the biomolecule and a dye as label, comprising the steps:
(a) providing a labelling solution comprising bioconjugates and non-reacted dye,
(b) adding a solid support to the labelling solution, wherein the non-reacted dye covalently couples to the solid support, and
(c) isolating the bioconjugates by separation, eg. by filtration or centrifugation.

In a preferred embodiment, the rylene dye used as label is a rylene diimide compound of formula (I) and, particularly preferably, a compound of formula (I), wherein n is 2 or 3. Further, the solid support is preferably a resin carrying polyethylene glycol with at least one terminal amino group.

The purification of the conjugates is accomplished by coupling the unreacted dye to the solid support being a resin. The resin consists of a low crosslinked polystyrene matrix onto which polyethylene glycol was grafted containing free terminal amino groups. Usually, such resins are employed for solid phase organic synthesis. Here, the non-reacted dye is covalently captured after addition of the support to the labelling solution, and the labeled enzyme is isolated by filtration or centrifugation. In contrast to the methods of the art using affinity chromatography, the resin which is functionalized with amino groups enables a chemical covalent reaction with the non-reacted dye. The use of such a covalent chemical binding reaction for a purification method of biomolecules has not been described in the prior art before. This novel strategy allows convenient and fast separation of labeled enzymes without the need to perform time-consuming chromatographic or size exclusion purification steps.

The invention is further illustrated by the following Figures and non-limiting Examples.

### FIGURES

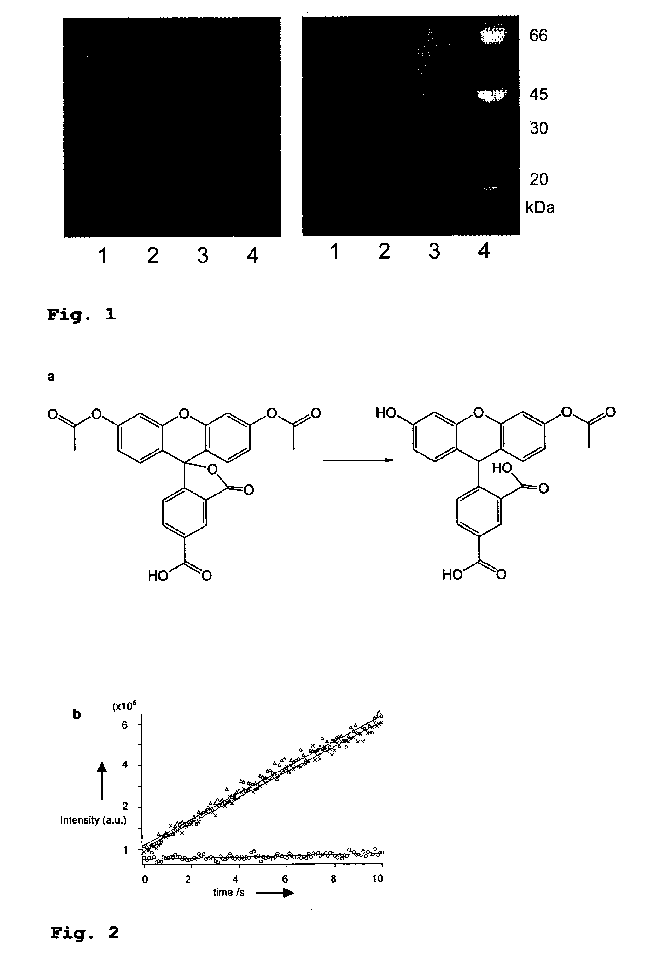

**Fig. 1****.** SDS-PAGE electrophoresis of PLA1 labeled with compound **5.** Panel A corresponds to UV transillumination visualization and Panel B was stained with Coomassie Blue. Lane 4 is the protein molecular weight standard. Lane 2 contains compound 5. Lane 1 is the phospholipase and lane 3 the labeled phospholipase.
**Fig. 2****.** Relative PLA1 enzymatic activity in bulk. Using a pro-fluorescent substrate, it is possible to follow the reaction kinetics through the increase in the fluorescent intensity; **(a)** Scheme of the hydrolysis of non-fluorescent 5-carboxyfluorescein diacetate (5-CDFA) yielding the fluorescent product 5-carboxyfluorescein (5-FAM). **(b)** Fluorescence intensity of a 5-FAM as a function of time. For the activation of the surface enzyme in solution (e.g. in order to open the lid protecting the active site), Triton X-100 was added to the solution. 5-CDFA: 0.7 mM; Triton X-100: 0.5 mM (X) non-labeled PLA1 (3E-8M), 3001.3±29.1 s⁻¹ (Δ) PLA1 labeled with N-hydroxysuccinimide perylene derivative **5** (cf. Scheme 1, Example 1) (3E-8M), 3083.1±38.1 s⁻¹ (o) 5-CFDA autohydrolysis 110.54±17.9 s⁻¹. From the Figure, it is shown that the labelling of the enzyme with NHS-PDI has no effect on its activity towards 5-CFDA.

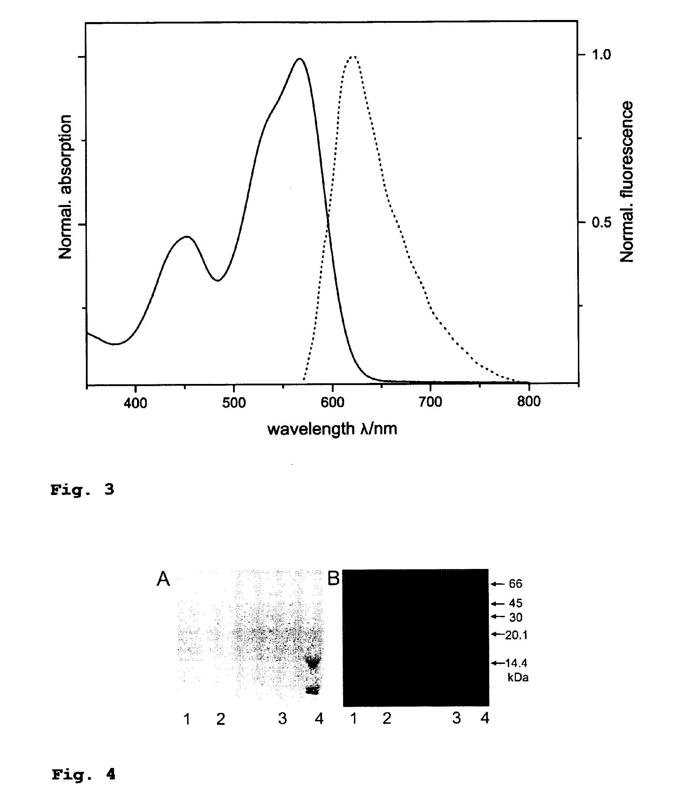

**Fig. 3****.** Normalized absorption (solid line) and emission spectra of the perylene thioester 9 in aqueous medium
**Fig. 4****.** SDS-PAGE electrophoresis of the PAI-1 labeled with compound **9** of scheme 2. Panel A corresponds to UV-transillumination visualisation. Panel B was stained with Comassie Blue. Lane 1 is the labelled PAI-1. Lane 4 contains compound **9.** Lane 3 is the N-terminal cysteine PAI-1 and lane 2 is a molecular weight marker.

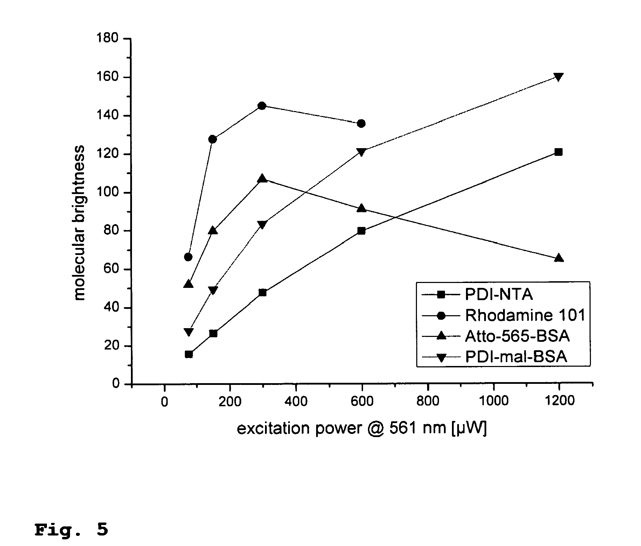

**Fig. 5****.** Relative molecular brightness of the NTA-functionalized rylene dye **10** of scheme 3, rhodamine 101, a maleimide-functionalized perylene dye (compound **3**) bound to BSA, and BSA-bound Atto 565

### EXAMPLE 1

### Synthesis of N-hydroxysuccinimide ester-functionalized perylene and terrylene labels

The synthesis of compounds **2, 3, 4, 5, 7** and **8** is shown in Scheme 1.

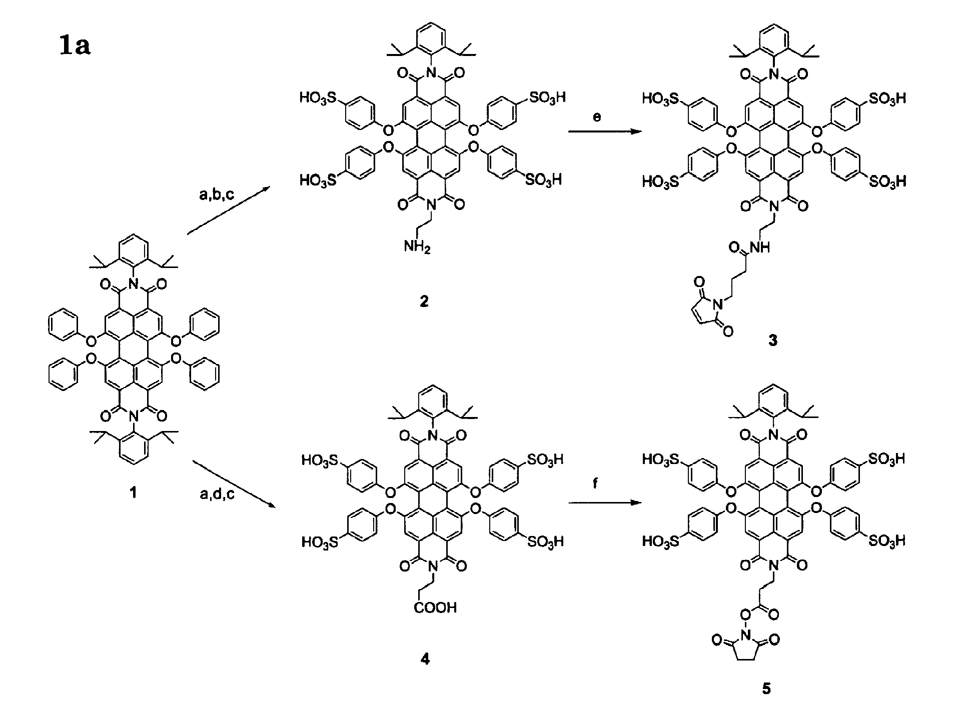

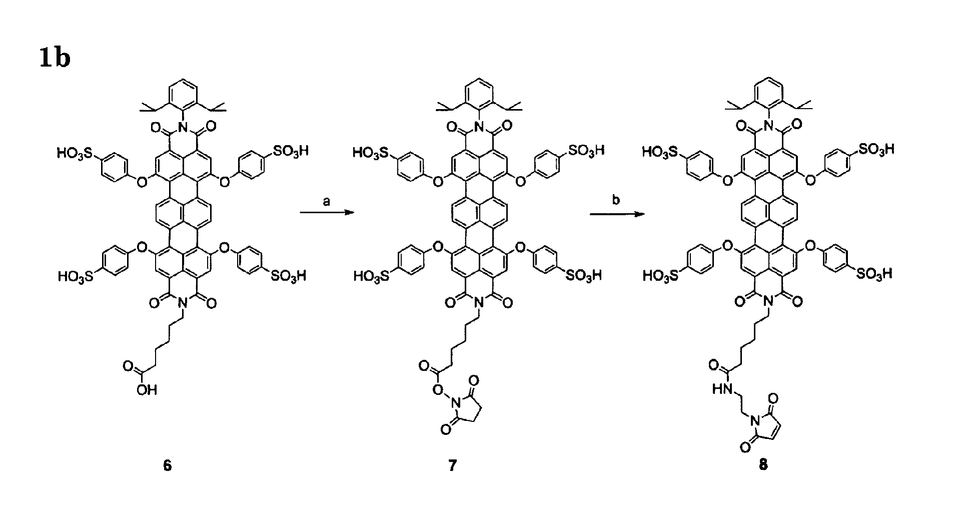

Scheme 1. Synthesis of perylene and terrylene labels. **1a)** Reagents, conditions and yields: a) KOH, isopropanol, 140°C, 12 h, 24%; b) ethylenediamine, toluene, 60°C, 3 h, 60%; c) H₂SO₄, 16 h, 97%; d) 3-aminopropanoic acid, propionic acid, 140°C°, 2 h, 65%; e) GMBS, triethylamine (TEA), DMF, 5 h, 95%; f) TSTU, DIPEA, DMF, water, 1,4-dioxane, 1 h, 85%. **1b)** Reagents, conditions and yields: a) TSTU, DIPEA, DMF, water, 1,4-dioxane, 1 h, 80%; b) 1-(2-aminoethyl)pyrrol-2,5-dione, DMF, TEA, 5 h, 90%.

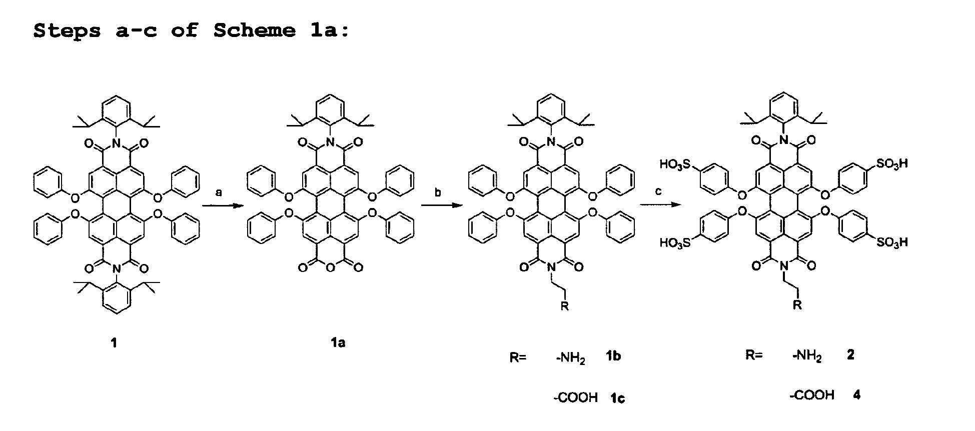

### N-(2,6-diisopropylphenyl)-1,6,7,12-tetraphenoxyperylene-3,4:9,10-tetracarboxy-9,10-monoanhydride-3,4-monoimide 1a:

To a solution of N,N'-bis(2,6-diisopropylphenyl)-1,6,7,12-tetraphenoxyperylene-3,4:9,10-tetracarboxydiimide (7.56 g, 7.00 mmol) in 2-propanol (1 L) was added KOH (148 g, 2.64 mol) dissolved in water (130 mL). The reaction was refluxed under argon at 110°C for 12 h. After cooling to room temperature the solution was poured into HCl/H₂O (60 mL/3.5 L). The precipitate was filtered and dried under vacuum at 60 °C. The solid was dissolved in acetic acid (100 mL) and refluxed at 80 °C for 30 min. Water (150 ml) was added; the precipitate was filtrated and purified over silica gel using dichloromethane as eluent. The product was obtained as a red solid in a yield of 24%. ¹H-NMR (250 MHz, DCM-d2 , 300 K): δ = 8.16 (s, 2 H), 8.15(s, 2 H), 7.47 (t, *J* = 7,2 Hz, 1H), 7.35 (d , 2H), 7.33-7.28 (m, 8H), 7.21-7.13 (m, 4 H), 7.03 - 6.97(m, 8H), 2.73-2.62 (sept, *J* = 6,8 Hz, 2H), 1.08 (d, J = 6,9 Hz, 12H); ¹³C-NMR (300 MHz, DCM-d2, 300 K): δ = 163.54, 160.19, 156.17, 155.60, 146.33, 130.50, 130.42, 128.98, 125.29, 125.17, 124.38, 123.70, 122.49, 120.56, 120.39, 120.33, 118.79, 29.43, 24.04 ppm; UV-Vis (CHCl₃) : λₘₐₓ (ε) = 443 (14100), 539 (23400), 577 (37300) nm; MS (FD): m/z (rel.int.) 920 [M+H]⁺; elemental analysis calc'd (%) for C₆₀H₄₁NO₉: C 78.33, H 4.49, N 1.52; found C 78.36, H 4.47, N 1.62

### N-(2,6-diisopropylphenyl)-N'-(4-aminoethyl)-1,6,7,12-tetraphenoxyperylene-3,4:9,10-tetracarboxydiimide 1b:

**1a** (200 mg, 0.2456 mmol) and 1,2-ethylenediamine (130 mg, 2.45 mol) were added to toluene (20 mL). The mixture was stirred under argon at 60°C for 3 h. Following the reduction of the solvent, the material was purified over silica gel using dichloromethane/acetone 9/1 and dichloromethane/ethanol 10/3 as eluent resulting in 1b as a red solid (60% yield). ¹H-NMR (250 MHz, DCM-d2, 300 K): δ = 8.16 (s, 2 H), 8.15(s, 2 H), 7.47 (t, *J* = 12.5 Hz, 1H), 7.30 (m, 10H), 7.14 (m, 4 H), 6.98 (m, 8H), 4.16 (t, J = 12 Hz, 2H), 2.95 (t, *J* = 12.5 Hz, 2H), 2.65( m, 2H), 1.08 (d, *J* = Hz, 7.5H, 12H); ¹³C-NMR (300 MHz, DCM-d2, 300 K): δ = 163.94, 163.83, 156.32, 156.14, 155.96, 146.55, 133.57, 131.62, 130.52, 129.95, 125.11, 125.08, 124.54, 123.37, 121.44, 121.16, 120.76, 120.63, 120.48, 120.44, 40.66, 31.16, 29.59, 25.34, 24.23 ppm; UV-Vis (CHCl₃): λₘₐₓ (ε) = 450 (12 348), 538 (21 708), 580 (35 991); Fluorescence (CHCl₃, excitation 566 nm) λₘₐₓ = 620 nm; MS (FD): m/z (rel.int.) 962 (100 %) [M+]; elemental analysis calc'd (%) for C₆₂H₄₇N₃O₈ : C 77.33, H 4.88, N 4.36; found C 76.87, H 4.54, N 4.00

### N-(2,6-diisopropylphenyl)-N'-(4-carboxyethyl)-1,6,7,12-tetraphenoxyperylene-3,4:9,10-tetracarboxydiimide 1c:

**1a** (500 mg, 0.59 mmol) and 4-aminopropionic acid (532 mg, 5.9 mmol mol) were added to propionic acid (50 mL). The mixture was stirred under argon at 140 °C for 3 h. After cooling the reaction mixture to room temperature, the product was precipitated with water (250 mL). The precipitate was filtered, washed with water and dried under vacuum. Further purification was done by column chromatography on silica gel with dichloromethane/acetone 9/1 and dichloromethane/ethanol 10/3 as eluent to afford 1c in a yield of 65 %. ¹H-NMR (250 MHz, DCM-d2, 300 K): δ = 8.06 (s, 4 H), 7.37 (t, *J* = 7.5 Hz, 1H), 7.19 (m, 10H), 7.03 (m, 4 H), 6.87 (d, *J* = 7.5 Hz, 8H), 4.26 (t, *J* = 10 Hz, 2H), 2.59 (m, 4H), 1.00 (d, *J* = 7.5 Hz, 12H); ¹³C-NMR (250 MHz, DCM-d2, 300 K): δ = 163.82, 156.37, 146.52, 130.50, 125.12, 125.06, 124.55, 123.35, 123.08, 121.33, 120.48, 31.99, 29.59, 24.22 ppm; UV-Vis (CHCl₃): λₘₐₓ (ε) = 450 (13 298), 536 (23 728), 580 (38 132); Fluorescence (CHCl₃, excitation 566 nm) λₘₐₓ = 620 nm; MS (FD): m/z (rel.int.) 990 (100 %) [M⁺]; elemental analysis calc' d (%) for C₆₃H₄₆N₂O₁₀xH₂O: C 74.92, H 4.78, N 2.78; found C 74.38, H 4.78, N 2.58

### N-(2,6-diisopropylphenyl)-N'-(4-aminoethyl)-1,6,7,12-tetra(4-sulfophenoxy)-perylene-3,4:9,10-tetracarboxydiimide 2:

**1b** (100 mg, 0.1 mmol) was added to concentrated sulfuric acid (3 mL). The flask was sealed, and the mixture was stirred at room temperature for 16 h. Water was slowly added to the flask and the resulted solution was dialyzed in water. The solution was freeze dried to give the product as a red solid in 97 % yield. ¹H-NMR (250 MHz, DMSO-d6, 300 K): δ = 7.97 (s, 2 H), 7.86 (s, 2 H), 7.67 (d, *J* = 7.5 Hz, 5H), 7.57 (d, *J* = 7.5 Hz, 5H), 7.41 (t, *J* = 12 Hz, 1H), 7.38 (d, *J* = 7.5, 2H), 7.01 (d, *J* = 7.5, 4H), 6.93 (d, *J* = 7.5, 4H), 3.08 (t, *J* = 7.5 Hz, 2H), 2.68 (sept, *J* = 10 Hz, 2H), 1.01 (d, *J* = 5 Hz, 12H) ;¹³C-NMR (250 MHz, DMSO-d6, 300 K): δ = 163.61, 163.34, 157.38, 157.22, 155.42, 155.07, 146.02, 141.20, 141.10, 132.84, 132.33, 130.59, 129.38, 128.15, 128.03, 123.79, 123.42, 123.19, 121.35, 121.08, 120.89, 120.36, 119.07, 38.80, 37.92, 29.07, 23.08 ppm; UV-vis (H₂O): λₘₐₓ (ε) = 450 (10 850), 534 (21 825), 560 (22 243) ; Fluorescence (H₂O, excitation 566 nm) λₘₐₓ = 620 nm; MS (MALDI-TOF): m/z (rel. int.) = 1283 (100 %) [M⁺]

### N-(2,6-diisopropylphenyl)-N'-(3-carboxyethyl)-1,6,7,12-tetra(4-sulfophenoxy)-perylene-3,4:9,10-tetracarboxydiimide 4:

**1c** (100 mg, 0.1 mmol) was added to concentrated sulfuric acid (3 mL). The flask was sealed, and the mixture was stirred at room temperature for 48 h. Water was slowly added to the flask and the resulting solution was dialyzed in water. The solution was freeze dried to give the product as a red solid in 97 % yield. ¹H-NMR (300 MHz, DMSO-d6, 300 K): δ = 7.92 (s, 2 H), 7.88 (s, 2 H), 7.66 (m, 4H), 7.60 (m, 4H), 7.40 (t, *J* = 12 Hz, 1H), 7.27 (d, *J* = 9 Hz, 2H), 6.98 (m, 8H), 4.19 (t, *J* = 12 Hz, 2H), 2.68 (sept, *J* = 10 Hz, 2H), 1.00 (d, *J* = 9Hz, 12H); ¹³C-NMR (300 MHz, DMSO-d6, 300 K): δ = 172.25, 162.51, 162.14, 155.26, 155.07, 154.78, 145.43, 144.86, 132.33, 132.03, 130.45, 127.72, 127.69, 123.59, 122.48, 119.87, 119.08, 118.99, 118.88, 31.99, 28.19, 23.64 ppm; UV-Vis (H₂O): λₘₐₓ (ε) = 450 (11 454), 534 (22 510), 564 (25 059); Fluorescence (H₂O, excitation 566 nm) λₘₐₓ = 622 nm; MS (MALDI-TOF): m/z (rel. int.) = 1311 (100 %) [M⁺]

### N-(2,6-diisopropylphenyl)-N'-[N-(4-maleimidobutyryl)-amino-ethyl]-1,6,7,12-1,6,7,12-tetra(4-sulfophenoxy)-perylene-3,4:9,10-tetracarboxydiimide 3:

To a solution of **2** (50 mg, 0.04 mmol) in dry DMF (3 mL) was added triethylamine (0.2 mL) and 4-maleimidobutyric acid N-succinimidyl ester (GMBS) (10 mg, 0.035 mmol). After 5 h the solvents were removed under vacuum. Dichloromethane (10 mL) was added and the solution filtered. The solvent was removed under vacuum and the product was obtained as a red solid in 95% yield. ¹H-NMR (300 MHz, DCM-d2, 300 K): δ = 8.15 (s, 2 H), 8.13 (s, 2 H), 7.76 (m, 8H), 7.45 (t, *J* = 12 Hz, 1H), 7.48 (d, *J* = 12 H, 2H), 6.98 (m, 8H), 6.59 (s, 2H), 4.27 (t, *J* = 12 Hz, 2H), 3.53 (m, 2H), 3.41 (t, *J* = 15 Hz, 2H), 2.68 (sept, *J* = 15 Hz, 2H), 2.06 (t, *J* = 15 Hz, 2H), 1.74 (m, 2H), 1.07 (d, *J* = 9 Hz, 12H); ¹³C-NMR (Spinecho, 300 MHz, DCM-d2, 300 K): δ = 173.34, 173.05, 171.50, 163.81, 163.62, 157.14, 157.08, 155.95, 146.58, 142.97, 142.84, 134.66, 133.44, 133.31, 131.52, 129.96, 128.67, 128.57, 124.54, 123.62, 121.55, 121.06, 119.83, 40.43, 38.65, 37.65, 33.78, 29.58, 26.05, 25.00, 24.25 ppm; UV-Vis (H₂O): λₘₐₓ (ε) = 450 (8 367), 534 (16 199), 566 (16 569); Fluorescence (H₂O, excitation 566 nm) λₘₐₓ = 620 nm; MS (MALDI-TOF): (100 %) [M⁺] 1447

### N-(2,6-diisopropylphenyl)-N'-[3-(N-succinimidyl)carboxyethyl]-1,6,7,12-tetra(4-sulfophenoxy)-perylene-3,4:9,10-tetra-carboxydiimide 5:

To a solution of **4** (50 mg, 0.038 mmol) in DMF (1 mL), dioxane (1 mL) and water (0.5 mL), was added diisopropylethylamine (DIPEA) (7.4 mg, 0.057 mmol) and N,N,N',N'-tetramethyl (succinimido) uronium tetrafluoroborate (TSTU) (14.3 mg, 0.0475 mmol). After 1 h the mixture was filtered, and the solvents removed under vacuum. Water (2 mL) was added and the solution freeze dried to give the product as red solid in 85% yield. ¹H-NMR (300 MHz, DMSO-d6, 300K): δ = 7.92 (s, 2 H), 7.88 (s, 2 H), 7.66 (m, 4H), 7.60 (m, 4H), 7.40 (t, *J* = 12 Hz, 1H), 7.27 (d, *J* = 9 Hz, 2H), 6.98 (m, 8H), 4.30 (t, *J* = 12 Hz, 2H), 2.76 (m, 4H), 2.68 (sept, *J* = 10 Hz, 2H), 1.00 (d, *J* = 9Hz, 12H); ¹³C-NMR (300 MHz, DMSO-d6, 300 K): δ =169.95, 166.79, 162.58, 162.23, 154.87, 145.50, 144.90, 132.42, 132.10, 130.52, 127.78, 123.67, 123.04, 122.60, 119.96, 199.40, 119.28, 118.97, 53.49, 41.73, 28.28, 25.40, 25.18, 23.72, 18.04, 16.70, 12.35 ppm; UV-Vis (H₂O): λₘₐₓ (ε) = 450 (9 951), 534 (18 867), 566 (21 071); Fluorescence (H₂O, excitation 566 nm) λₘₐₓ = 620 nm; MS (MALDI-TOF): 1407 (100 %) [M⁺]

### Scheme 1b

### N-(2,6-diisopropylphenyl)-N'-[5-(N-succinimidyl)carboxypentyl]-1,6,9,14-tetra(4-sulfonylphenoxy)-terrylene-3,4,11,12-tetracarboxidiimide 7:

To a solution of **6** (100 mg, 0.067 mmol) in DMF (1 mL), dioxane (1 mL) and water (0.5 mL), was added diisopropylethylamine (12 mg, 0.1 mmol) and TSTU (25 mg, 0.083). After 1 h the mixture was filtered, and the solvents removed under vacuum. Water (2 mL) was added and the solution was freeze dried to give the product **7** as a blue-green solid in 80% yield. ¹H-NMR (700 MHz, DMSO-d6, 300 K): δ = 9.37 (s, 4H), 8.03 (s, 2H), 7.95 (s, 2H), 7.70 (d, *J* = 6.3 Hz, 4H), 7.67 (d, *J* = 6.3 Hz, 4H), 7.42 (t, *J* = 9 Hz, 1H), 7.29 (d, *J* = 9 Hz, 2H), 7.22 (m, 8H), 3.93 (m, 2H), 2.65 (sept., *J* = 13 Hz, 2H), 2.17 (t, *J* = 7.27 Hz, 2H), 1.58 (m, 2H), 1.50 (m, 2H), 1.29 (m, 2H), 1.01 (d, *J* = 6 Hz, 12H); ¹³C-NMR (500 MHz, DMSO-d6, 300 K): 174.03, 168.53, 166.25, 162.22, 155.03, 153.60, 145.32, 144.69, 144.61, 130.52, 128.35, 128.22, 127.76, 125.36, 124.35, 123.48, 122.70, 121.95, 121.53, 118.28, 118.13, 40.33, 33.24, 30.99, 30.96, 28.19, 26.83, 25.74, 23.93, 23.49 ppm; UV-Vis (H₂O): λₘₐₓ (ε) = 426 (4 569), 638 (24 468); MS (MALDI-TOF): 1575 (100 %) [M⁺]

### N-(2,6-diisopropylphenyl)-N'-[5-(N-(2-(4-maleimidobutyryl)-ethyl)-carboxypentyl]-1,6,9,14-tetra(4-sulfonylphenoxy)-terrylene-3,4,11,12-tetracarboxidiimide 8:

To a solution of **7** (100 mg, 0.063 mmol) in dry DMF (3 mL) was added triethylamine (6.36 mg, 0.063 mmol) and 2-maleimidoethylamine trifluoroacetate salt (15 mg, 0.059 mmol). After 5 h the solvent was removed under vacuum. Dichloromethane (10 mL) was added and the solution filtered. The solvent was removed under vacuum and the product was obtained as a blue-green solid in 90% yield. ¹H-NMR (700 MHz, DCM-d2, 300 K): 9.15 (s, 4H), 8.19 (s, 2H), 7.82 (m, 8H), 7.46 (t, *J* = 9 Hz, 1H), 7.31 (d, *J* = 9 Hz, 2H), 7.15 (m, 8H), 7.03 (s, 2H), 3.91 (m, 2H), 3.36 (m, 4H), 2.65 (sept., *J* = 13 Hz, 2H), 2.17 (t, *J* = 7.27 Hz, 2H), 1.58 (m, 2H), 1.50 (m, 2H), 1.29 (m, 2H), 1.08 (d, *J* = 6 Hz, 12H); ¹³C-NMR (700 MHz, DCM-d2, 300 K): 173.28, 171.53, 163.49, 162.89, 161.47, 161.28, 157.46, 157.42, 154.44, 154.42, 146.78, 143.21, 134.7, 129.03, 128.96, 128.94, 119.15, 116.86, 38.05, 37.98, 36.56, 30.29, 29.67, 26.08, 24.39 ppm; UV-Vis (H₂O): λₘₐₓ (ε) = 426 (2 782), 638 (16 633); MS (MALDI-TOF): 1665 (100 %) [M⁺+ 3Na⁺]

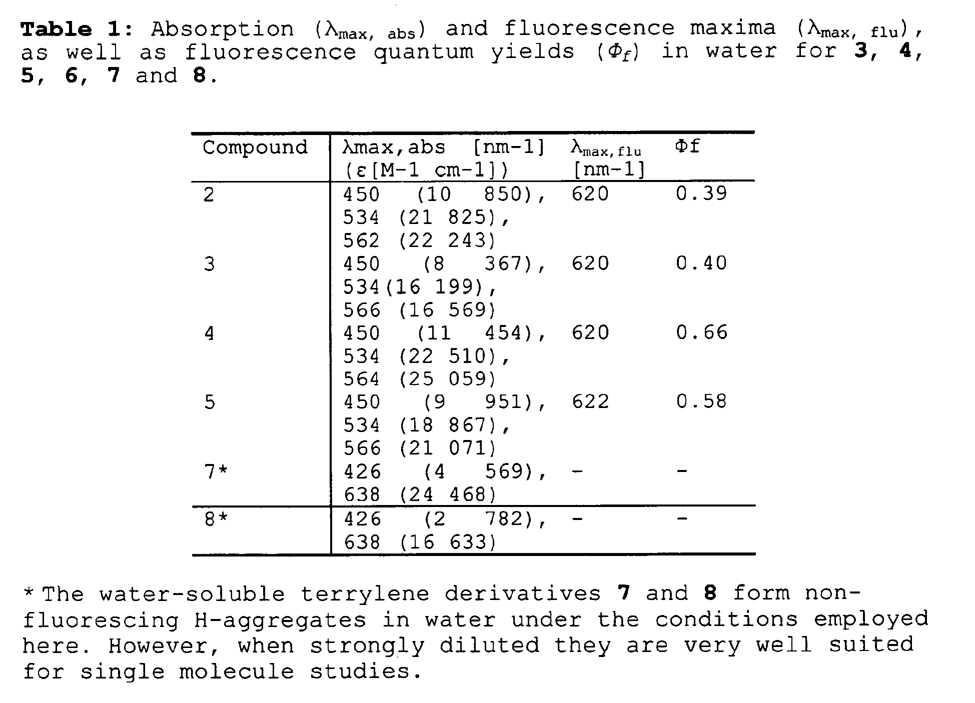

### EXAMPLE 2

### Fluorescent labelling of phospholipase (PLA 1) with 5

Phospholipase A1 (2.56 mg) in 10 mM succinic acid, 0,4M NH₄SO₄, pH 6 (400 µL) was reacted at 4 °C for one hour with **5** (1.5 mg) freshly dissolved in 0.01 M NaHCO₃. TentaGel HL NH₂ resin (Rapp Polymere GmbH) (20 mg) was added and the solution shaken for 5 min. The mixture containing the polymer resin was applied to a spin column and filtered. The colored solution containing the labeled enzyme was collected and quantified by 10% SDS-PAGE and by fluorescence correlation spectroscopy (FCS). The solution of the labeled phospholipase in 0.01 M NaHCO₃ was stored at -80°C.

### EXAMPLE 3

### Test of the bulk enzymatic activity of PLA1 labeled with 5

PLA1 catalyses the saponification of the acetate groups of the fluorescein derivative that is non-fluorescent (see Fig. 2a). The hydrolysis of the substrate can be followed via the increase in the fluorescence intensity due to the formation of the fluorescent product upon the enzymatic reaction. Fig.2B shows fluorescence intensity changes of a similar solution of pro-fluorescent substrate as a function of time without the enzyme (0), after adding non-labeled enzyme (X), and after adding the labeled enzyme (Δ), respectively. The rate constants of the hydrolysis were estimated to be 3001.3±29.1 (s⁻¹) and 3083.1±38.1 (s⁻¹) for the labeled enzyme and the non-labeled enzyme, respectively. The autohydrolysis of 5-CFDA was measured as 110±17.9 (s⁻¹). The result clearly shows that no activity was lost by the labelling.

The fluorescence intensity was measured using SPEX FLUOROLOG 1500 (Spex Industries, Metuchen, NJ). The time base scan experiments were performed with 1s integration time. The fluorescent product was excited at 500nm. The fluorescent emission was detected at 520nm.

### EXAMPLE 4

### Synthesis of a water-soluble perylene dye functionalized with a thioester group

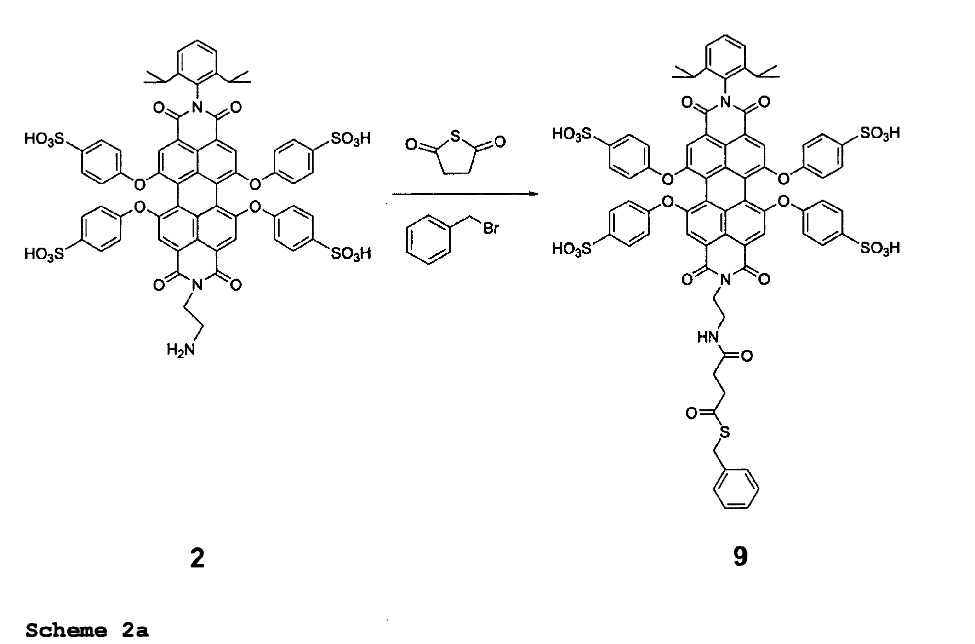

To a solution of N-(2,6-diisopropylphenyl)-N'-(4-aminoethyl)-1,6,7,12-tetra(4-sulfophenoxy)-perylene-3,4:9,10-tetracarboxydiimide **2** (50 mg, 0.04 mmol) in N-methyl pyrrolidinone (NMP) was added thiolane-2,5-dione (6 mg, 0.052 mmol), followed by N,N-diisopropylethylamine (DIPEA) (10 mg, 0.08 mmol). After one hour, the reaction mixture was diluted with 100 mM NaOAc buffer (pH 5.2) and cooled to 4°C. Benzyl bromide (14 mg, 0.08 mmol) was added with thorough mixing and additionally react for 10 minutes. The product was purified using a Sep-Pak C18 cartridge (Millipore; pre-washed with 10 mL acetonitrile and 10 mL water; washed with 20 mL water; eluted with 1 mL 60% methanol) and dried. The product was isolated as red solid in 50 % yield. ¹H-NMR (250 MHz, DMSO-d6, 300 K): δ = 7.95 (s, 2 H, Ar**H**), 7.91 (s, 2 H, Ar**H**), 7.62 (m, 8H, Ar**H**), 7.40 (t, *J* = 7.5 Hz, 1H, Ar**H**), 7.29 (d, J = 7.5, 6H, Ar**H**), 6.99 (d, *J* = 7.5, 4H, Ar**H**), 6.96 (d, *J* = 7.5, 4H, Ar**H**), 4.16 (s, 2H, C**H**₂SCO), 3.58 (t, *J* = 12.5, 2H, C**H**₂N), 3.08 (t, J = 15 Hz, 2H, C**H**₂NH), 2.68 (sept, *J* = 12.5 Hz, 2H, C**H**(CH₃)₂), 2.28 (t, *J* = 12.5 Hz, 2H, C**H**₂CO), 2.14 (t, *J* = 12.5 Hz, 2H, C**H**₂CO), 1.00 (d, *J* = 5 Hz, 12H, C**H**₃); ¹³C-NMR (250 MHz, DMSO-d6, 300 K): δ = 178.14 (SC=O), 174.02 (NH**C**=O), 171.55 (**C**=O), 162.94 (**C**=O), 155.48, 155.27, 145.84, 145.24, 132.80, 132.43, 130.87, 128.14, 124.04, 123.69, 122.86, 120.33, 119.33, 53.75 (**C**H₂S), 48.79 (**C**H₂N), 42.07 (**C**H₂N), 36.52 (**C**H₂CO), 30.42 (**C**H₂CO), 24.08 ppm. UV-vis (H₂O): λₘₐₓ (ε) = 452 (10 643), 536 (19 080), 568 (23 401); Fluorescence (H₂O, excitation 568 nm) λₘₐₓ = 622 nm;

In contrast to the above protocol, applying the procedure described by Schule and Pannel (Bioconjugate Chemistry 13, 1039-1043) for conversion of a succinimidyl ester of the chromophore via activation of benzyl mercaptane with trimethylaluminium did not lead to the formation of the target compound **9.**

The obtained perylene thioester **9** is excellently soluble in water and exhibits an absorption maximum at 568 nm (ε = 23 400 M⁻¹ cm⁻¹) and an emission maximum at 622 nm. The perylene thioester **9** shows high fluorescence quantum yield (Φ*_{f}*) of 54 % in aqueous medium measured at room temperature using Cresyl Violet in methanol as a reference.

The FCS analysis of perylene-thioester showed only half the molecular brightness compared to a single sulforhodamine B. However the extinction coefficient of the perylene is only one third of sulforhodamine B at laser excitation wavelength of 561 nm. Accordingly the molecular brightness of a single perylene-thioester as measured by FCS indicates a fluorescence quantum yield similar to sulforhodamine B. After binding to PAI-1 (Example 5) the brightness of the perylene dye slightly decreased by 15 percent. This indicates an almost independent fluorescence quantum yield from the local protein environment, which makes the perylene dye a very attractive new label for N-terminal cysteines in proteins.

### EXAMPLE 5

### Site-specific-labelling of PAI-1 with 9

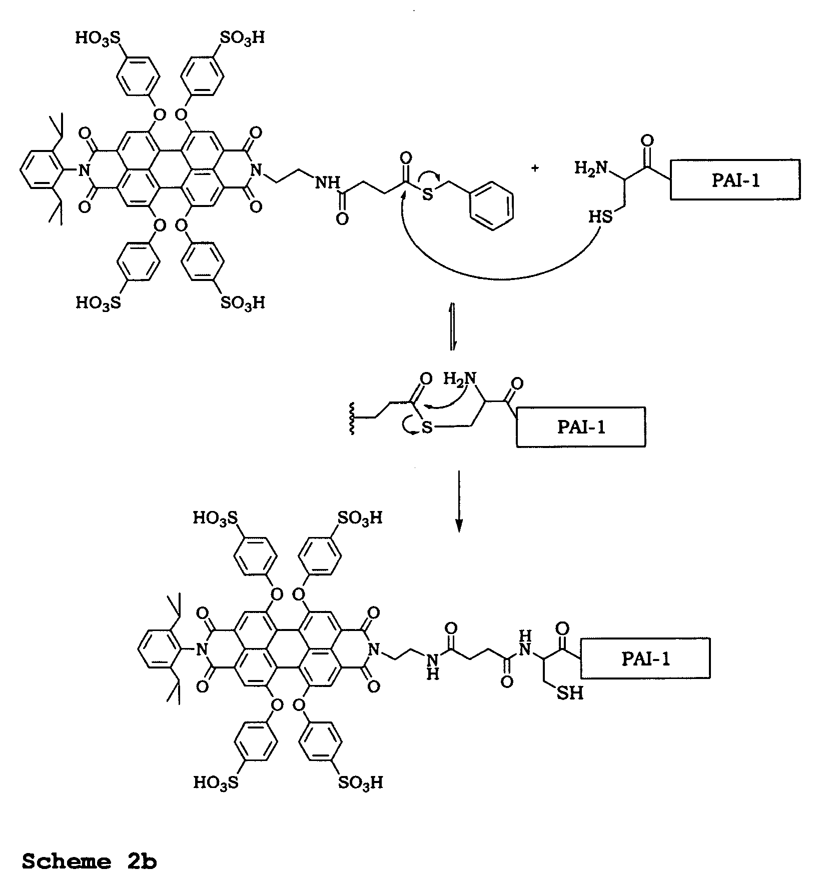

To prove the suitability of **9** for protein labelling, the chromophore was attached to plasminogen activator inhibitor-1 (PAI-1) stable mutant, containing only one cysteine residue at the N-terminus. The PAI-1 is a member of the SERine Protease Inhibitor superfamily (SERPINS) and exhibits a molecular weight of 43 kDa. The N-terminal cysteine containing PAI-1 was chosen as model protein in this study. The method and conditions applied for the labelling of the PAI-1 are the same as described by Dawson et al. (Science 266, 776-779; 1994), which are often referred to as "standard native chemical ligation conditions". These strongly denaturing conditions ensure that the reactivity of the N-terminal cysteine is independent from the three-dimensional structure of the protein in its native state.
Under these conditions transesterification of the thioester **9** with the cysteine thiol of the protein occurs, followed by an S-N acyl shift to generate the conjugate that exhibits a stable amide bond (Scheme 2b).

The N-terminal cysteine PAI-1, stable mutant was dissolved in 100 mM phosphate buffer, containing 6 M Gn·HCl, 4% v/v thiophenol and 100 mM sodium phosphate at pH 7.6 to a final protein concentration of 2 mM. Equimolar amounts of the dye **9** were dissolved in the same buffer and added to the protein solution and the reaction mixture was allowed to react for 24 hours at room temperature. The reaction mixture was analyzed using SDS-PAGE electrophoresis.

### Refolding of conjugate

Denatured Perylene-PAI-1 conjugate was refolded by dialysis (2 h, 4°C) in 20 mM Na-acetate, 1 M NaCl, 0.01% Tween-80 (v/v), pH 5,6 followed by a second dialysis step (overnight, 4°C). The protein was subsequently concentrated using centrifugal filter devices. The successful refolding of the Perylene-PAI-1 conjugate was confirmed by fluorescence correlation spectroscopy (FCS) analysis of the protein solution.

### EXAMPLE 6

### Synthesis of a water-soluble perylene dye functionalized with a nitrilotriacetic acid group

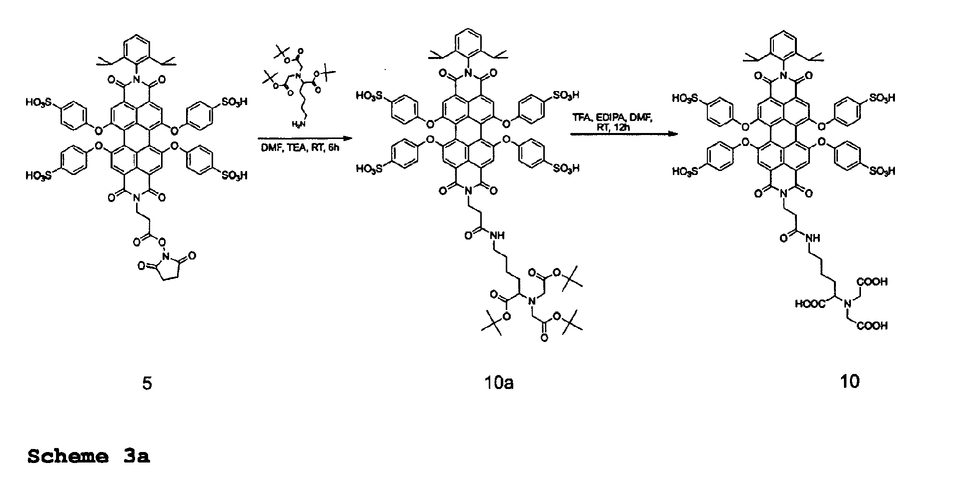

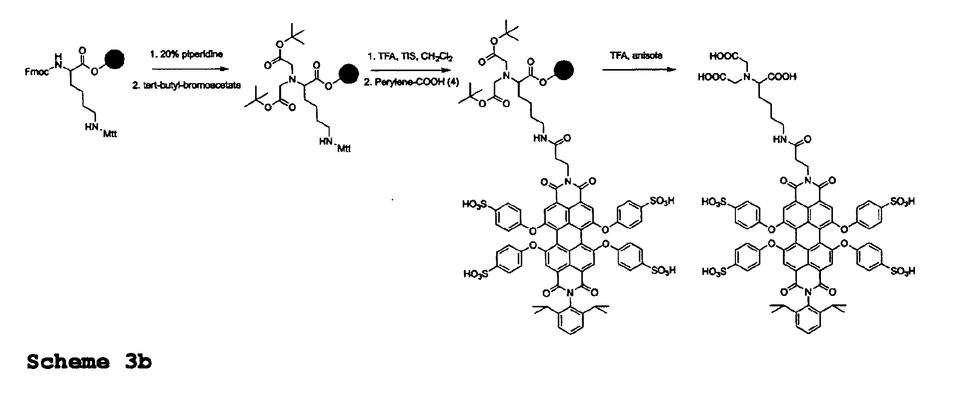

Two synthetic strategies were used to attach the NTA moiety to the perylene chromophore. One is based on solution phase synthesis while the other relies on a solid-phase approach.

In the first route, the water-soluble perylene N-hydroxysuccinimide ester **5** was reacted with t-butyl protected nitrilotriacetic acid to give the intermediate **10a.** After removal of the protective groups, the perylene dye **10** containing the NTA unit was obtained (Scheme 3a).

The second approach is based on a straightforward solid-phase synthesis of a nitrilotriacetic acid moiety as developed by Meredith et al. (Bioconjugate Chem. 2004, 15, 969-982). The NTA unit was conveniently coupled to a carboxyl functionalized perylene derivative by amide bond formation (Scheme 3b).

For both routes water-soluble perylene NTA derivative **10** was purified by size exclusion chromatography and obtained in 70% and 15% yield for the solution and solid-phase synthesis, respectively. The new chromophore has an absorption maximum at 562 nm (ε = 35 000 M⁻¹ cm⁻¹). The emission maximum is located at 615 nm. A fluorescence quantum yield of 15 % for **10** was determined by using Cresyl Violet in methanol as reference. When the same quantum yield measurement was carried out with the Ni²⁺ complexes of **10** no decrease in the fluorescence was observed. In addition the absorption and emission maxima remained unchanged.

In contrast to all NTA-modified fluorophores of the prior art, the above NTA-modified perylene dye **10** contains a much shorter spacer between the dye and the NTA (only two methylene groups). Remarkably, the photophysical properties of the chromophore remain unchanged upon Ni²⁺ binding. This new fluorescent reporter has a relatively low molecular weight (1555 g/mol), which makes it a good candidate for labelling proteins at the same time, avoiding the limitation of the large size of autofluorescent proteins or quantum dots. Protein labelling with the NTA functionalized perylene was successfully demonstrated using His-tagged ATP synthase (Example 7).

### Solution phase synthesis (Scheme 3a)

### N-(2,6-diisopropylphenyl)-N'-[3-(6-(tert-butyl 2,2'-(1-tert-butoxy-1-oxohexan-2-ylazanediyl)diacetate)carboxyethyl]-1,6,7,12-tetra(4-sulfophenoxy)-perylene-3,4:9,10-tetracarboxydiimide (10a)

N-(2,6-diisopropylphenyl)-N'-[3-(N-succinimidyl)carboxyethyl]-1,6,7,12-tetra(4-sulfo phenoxy)-perylene-3,4:9,10-tetracarboxydiimide (50 mg, 0.035 mmol) and diisopropyl ethylamine (DIPEA) (7.4 mg, 0.057 mmol) were added to a solution of N-(5-amino-1-carboxypentyl)iminodiacetic acid, tri-t-butylester (22.8 mg, 0.053 mmol) in dry DMF (3 mL). The reaction vessel was purged with argon and stirred for 16 hours at room temperature. Ethylacetate (15 mL) was added to the reaction mixture to obtain a pink precipitate, which was filtered. The precipitate was washed first with dichloromethane and then three times with ethyl acetate (3x 40 mL) and dried under vacuum to give the product as red solid in 70 % yield. ¹H-NMR (300 MHz, DMSO-d6, 300K): δ = 7.90 (s, 2 H), 7.88 (s, 2 H), 7.63 (m, 8H), 7.39 (t, *J* = 15 Hz, 1H), 7.29 (d, *J* = 9, 2H), 6.97 (m, 8H), 4.14 (t, *J* = 15 Hz, 2H), 3.56 (m, 2H), 3.32 (m, 4H), 3.17 (m, 2H), 2.95 (m 2H), 2.77 (m, 4H), 2.72 (m, 2H), 2.64 (m, 2H),1.35 (s, 27H), 1.00 (d, *J* = 9Hz, 12H); ¹³C-NMR (300 MHz, DMSO-d6, 300 K, Spinecho): δ = 171.89, 170.39, 162.97, 162.55, 155.72, 155.27, 155.21, 145.46, 128.17, 123.55, 122.88, 119.37, 80.30, 40.52, 32.56, 28.07, 22.58 ppm;

### N-(2,6-diisopropylphenyl)-N'-[3-(N-(5-amino-1-carboxypentyl)iminodiacetyl)carboxyethyl]-1,6,7,12-tetra(4-sulfophenoxy)-perylene-3,4:9,10-tetracarboxy diimide (10)

To a solution of **10a** (40 mg, 0.023 mmol) in dry DMSO (2 mL) were added triisopropylsilane (1 mL) and trifluoroacetic acid (TFA). After stirring the reaction mixture for 3 hours at room temperature, the volatiles were evaporated under reduced pressure. Water (5 mL) was added and the solution applied on a column fill with Biogel P30. The product was eluted with water and freeze dried to give **10** as red solid in 95% yield. ¹H-NMR (700 MHz, CD₃OD, 300K): δ = 8.55 (s, 2 H), 8.53 (s, 2 H), 8.18 (m, 8H), 7.76 (t, *J* = 14 Hz, 1H), 7.64 (d, *J* = 7, 2H), 7.43 (m, 8H), 4.54 (t, *J* = 14 Hz, 1H), 3.87 (m, 4H), 3.49 (m, 3H), 3.32 (m, 4H), 2.70 (m, 2H), 2.28 (m, 2H), 1.83 (m, 2H), 1.68 (t, *J* = 14 Hz, 2H), 1.47 (d, *J* = 7,12H); ¹³C-NMR (700 MHz, CD₃OD, 300K): δ = 168.33, 166.58, 158.54, 157.94, 156.52, 156.33, 152.51, 152.47, 149.49, 149.47, 141.82, 138.27, 138.26, 129.84, 124.08, 119.69, 114.20, 113.58, 111.91, 43.04, 38.03, 25.34, 24.71, 21.13, 19.44 ppm; UV-Vis (H₂O): λₘₐₓ (ε) = 448 nm (16 120), 534 nm (31 224), 562 nm (35 000) Fluorescence (H₂O, excitation 562 nm) λₘₐₓ = 614 nm ; MS (MALDI-TOF): 1599.51 (100 %) [M+2Na]

### Solid phase synthesis of Perylene-NTA (Scheme 3b)

All amine deprotection and coupling steps were monitored with ninhydrine. In a 25 mL manual peptide synthesis reaction vessel, Tenta TGR resin (2 g, 0.64 mmol) was swollen in dichloromethane (DCM) for 1 h. Fmoc-Lys(Mtt)-OH (1.15 g, 1.84 mmol), 1-Hydroxybenzotriazole hydrate (HOBt) (248 mg, 1.84), O-(Benzotriazol-1-yl)-N,N,N',N'-tetramethyluronium hexafluorophosphate (HBTU) (683 mg, 1.80 mmol), diisopropylethylamine (DIPEA) (237 mg, 1.84 mmol) was dissolved in dry dimethylformamide (DMF) (6.5 mL) added and shaken at 350 rpm at 25°C. After 12 h the resin was washed extensively with DCM. Fmoc deprotection was effected by six successive 15 min treatments of the resin with 20% piperidine in DCM. Afterwards the resin was washed extensively with DCM. To the washed resin was added a 20-fold excess of *tert*-butyl-bromoacetate (1.79 g, 9.2 mmol) with an equal amount of DIPEA (1.18 g, 9.2 mmol) and DCM (2 ml). The reaction vessel was shaken at 350 rpm at 45°C for 27 h. Fresh reagents were added and the vessel was further incubated at 45°C with shaking for 18 h. The beads were washed successively with DCM, ethyl acetate, and DCM again. The Mtt group was removed by five 3-min treatments with 10 mL of 1% TFA, 5% triisopropyl silane (TIS) in DCM. The beads were again washed extensivly with DCM. N-(2,6-diisopropylphenyl)-N-(4-carboxyethyl)-1,6,7,12-tetrasulfophenoxy-perylene-3,4:9,10-tetracarboxydiimide (100 mg, 0.076 mmol), HOBt (10 mg, 0.076), HBTU (50 mg, 0.074) and DIPEA (10 mg, 0.076) in dry DMF (0.5 mL) were added to the beads (86 mg, 0.02 mmol) in 2 mL of DMF. The beads were allowed to react with agitation for 24 h at 25°C. After washing of the sample, an equivalent amount of fresh reagents were added and allowed to react for 12 more hours. The beads were washed successively with DCM, ethyl acetate, methanol, DCM again, and dried briefly under vacuum. The product was cleaved from the resin with several 5-min treatments with 10 mL of 95% TFA in DCM. The material was reduced to dryness with rotary evaporation, precipitated with ice-cold diethyl ether, and dried under high vacuum.

The dried off-red material was dissolved in water and purified by size-exclusion chromatography using BioGel as stationary phase and water as eluent. The yield, relative to the amount of Fmoc-Lys(Mtt)-OH was 15%.

### Ni²⁺:NTA-Perylene

NiCl₂ (30 µl, 2.5x10⁻⁴ M/l solution in 0.01 N HCl) was added to Perylene NTA (1 ml, 5x10⁻⁵ M/l in water), and the solution was brought to pH 7 by addition of 0.8 mL 50 mM sodium acetate (pH 7), 200 mM NaCl. Following reaction for 30 min at 25°C, the product was purified using a Sep-Pak C18 cartridge (Millipore; pre-wahsed with 10 mL acetonitrile and 10 mL water; washed with 20 mL water; eluted with 1 mL 60% methanol) and dried.

### Fluorescence correlation spectroscopy (FCS)

FCS measurements were carried out on a confocal setup based on an Olympus IX71 inverted microscope. A solid-state laser (JIVE, Cobolt AB, Stockholm, Sweden) with emission at 561 nm was used and attenuated to 150 µW before focussing into the buffer solution by a water immersion objective (40x, N.A. 1.15, Olympus). The solution was placed on a microscope coverslide as a droplet of 25 to 50 µl. Scattered laser light was blocked by a dichroic beam splitter (DCXR 575, AHF, Tübingen, Germany) and out-of-focus fluorescence was rejected by an 100 µm pinhole in the detection pathway. Fluorescence was collected in the spectral range from 575 to 648 nm using an interference filter (AHF). Single photons were detected by an avalanche photodiode (SPCM AQR-14, Perkin Elmer) and registered by a TCSPC device (PC card SPC-630, Becker & Hickl, Berlin, Germany) for software calculation of the autocorrelation functions, *g⁽²⁾(t_{c})*.

Fluorescence correlation spectroscopy measurements were used to monitor the binding of the perylene dye to F₁-ATPase in solution. Confocal laser excitation at 561 nm was used to match the absorbance maximum of the perylene dye. Different concentrations of F₁-ATPase were pre-mixed with 1 nM perylene-Ni-NTA. As a control, the dye was mixed with F₁-ATPase in the absence of Ni²⁺. Increasing amounts of F₁-ATPase resulted in increasing diffusion times of the perylene-Ni-NTA due to conjugate formation with the His tagged protein. In the absence of Ni²⁺, no evidence for binding of the perylene-NTA dye was found by FCS measurements.

FCS was also used to determine the mean photon count rate or 'molecular brightness' of a single perylene-NTA in the absence of Ni²⁺ (Figure 5). The conventional dyes Rhodamine 101 and Atto-565 were used as references. For Rhodamine 101, a maximum fluorescence of about 145 000 counts/s per molecule saturated at an excitation power of 300 µW at the back aperture of the microscope objective was detected. At higher excitation power, photobleaching and increased population of the non-fluorescent triplet state reduced the brightness per molecule. Similar saturation behaviour was observed for Atto-565 which was bound to bovine serum albumin (BSA). In contrast, the molecular brightness of the perylene-NTA steadily increased up to an excitation power of 1.2 mW reaching 120 kHz and supporting the high photostability of the perylene chromophore. At this high laser power, the triplett yield was only 11 percent compared to about 45 percent for Atto-565 bound to BSA. In the presence of Ni²⁺, the relative brightness of the perylene derivative was reduced to about 77 percent. Binding of the perylene-Ni-NTA to the His tags of F₁-ATPase did not reduce the molecular brightness further but rather increased slightly. These results demonstrate the favourable properties of the new NTA-derivatized rylene dye and suggest its use for site-specific labelling of proteins.

### EXAMPLE 7

### Site-specific labelling of the His₆ tagged F₁ complex of ATP Synthase

To prove the suitability of **10** for protein labelling, the Ni-complex of the chromophore was incubated with His₆-tagged F₁ complex of FₒF₁-ATP synthase from *Escherichia coli*. The F₁ complex with subunit composition α₃β₃γδε contained three His₆ tags, one at the N-terminus of each of the β subunits. In order to detect the binding of the perylene dye to this protein in solution, fluorescence correlation spectroscopy experiments were carried out. Confocal laser excitation at 561 nm was used to match the absorbance maximum of the perylene dye. Different concentrations of F₁-ATPase were pre-mixed with 1 nM perylene-Ni-NTA. As a control, the dye was mixed with F₁-ATPase in the absence of Ni²⁺. Increasing amounts of F₁-ATPase resulted in increasing diffusion times of the perylene-Ni-NTA due to conjugate formation with the His tagged protein. Using a two-component model for bound and un-bound dye to fit the autocorrelation functions of the FCS experiments, a dissociation constant K_{D} = 3±1 µM was obtained for the perylene-Ni-NTA. In the absence of Ni²⁺, no evidence for binding of the perylene-NTA dye was found by FCS measurements.

### EXAMPLE 8

### Purification of biomolecules

The N-hydroxysuccinimide perylene derivative **5** was used for the labelling of phospholipase (PLAI) (Example 2). The purification of the resulting conjugates was accomplished by coupling the unreacted dye to a solid support. The resin consisted of a low crosslinked polystyrene matrix, onto which polyethylene glycol was grafted containing a free terminal amino group. Usually, such resins are employed for solid phase organic synthesis. Here, the non-reacted dye was covalently captured after addition of the support to the labelling solution, and the labeled enzyme was isolated by filtration. The gel electrophoretic analysis (Fig. 1) and fluorescence correlation spectroscopy measurements (data not shown) confirmed that the unreacted label was removed very efficiently. Similar results were obtained with TDI derivative **8.** This novel strategy allows convenient and fast separation of labeled enzymes without mthe need to perform time-consuming chromatographic or electrophoretic purification steps.

## Claims

1. A water-soluble rylene diimide dye of formula (I) 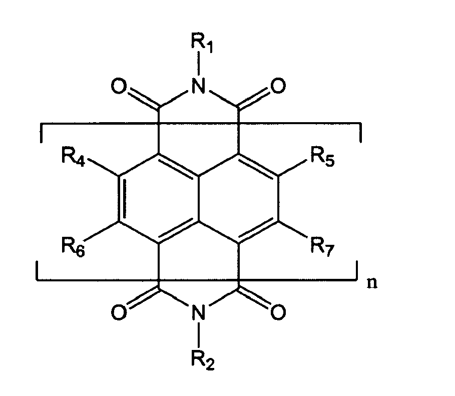 wherein R₁ is an organic residue which is attached to the nitrogen atom via a carbon atom,
n is an integer from 1 to 6,
R₂ is -(CH₂)ₘ-A-(CH₂)ₒ-B,
m is an integer from 0 to 20,
o is an integer from 0 to 20,
A is a linking group,
B is a coupling group selected from the group consisting of isothiocyanate, activated ester, carbonyl azide, sulfonyl chloride, aldehyde, alkyl halide, haloacetamide, maleimide, nitrilotriacetic acid, disulfide, thiosulfate, thiol and thioester,
and R₄, R₅, R₆ and R₇ are independently hydrogen or an organic residue, which carries at least one negatively or positively charged group in neutral medium or at least one hydrophilic uncharged group, wherein at least two of R₄, R₅, R₆ and R₇ are an organic residue, which carries at least one negatively or positively charged group in neutral medium or least one hydrophilic uncharged group.

2. Water-soluble rylene dye acording to claim 1, wherein n is an integer from 2 to 4.

3. Water-soluble rylene dye acording to claim 1 or 2, wherein the coupling group B is an activated ester, e.g. an N-hydroxysuccinimide ester.

4. Water-soluble rylene dye according to any one claims 1-3,
wherein the coupling group B is a maleimide, nitrilotriacetic acid or a thioester or thiol.

5. Water-soluble rylene dye according to any one of claims 1-3, wherein R₁ is an aliphatic residue or cyclic residue.

6. Water-soluble rylene dye according to any one of claims 1-4, wherein R₁ is an aromatic or heteroaromatic residue, in particular phenyl-, pyridyl- or naphthyl residue, which optionally contains one or more substituents.

7. Water-soluble rylene dye according to any one of claims 1-6, wherein at least two of R₄, R₅, R₆ and R₇ are an organic residue which carries a group selected from a quaternary ammonium group, an N-alkyl-pyridinium, N-alkyl-quinolinium or N-alkyl-isoquinolinium group, or SO₃H, COOH, SO₃M and COOM, wherein M is a cation, or a hydroxyl group and polyoxy-C₂-C₄-alkyl groups.

8. Water-soluble rylene dye according to any one of claims 1-7, wherein at least two of R₄, R₅, R₆ and R₇ comprise aromatic residues, in particular phenyl residues.

9. Water-soluble rylene dye according to any one of claims 1-8, wherein at least two of R₄, R₅, R₆ and R₇ have the formula
- O - Ar (III)
wherein Ar is an aromatic residue which contains at least one charged group.

10. Water-soluble rylene dye according to claim 9 wherein at least two of R₄, R₅, R₆ and R₇ have the formula (III) 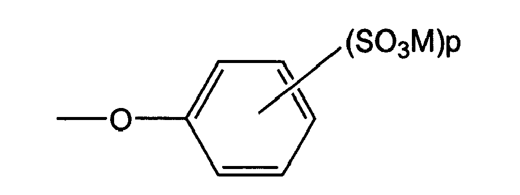 wherein M is a cation and p is an integer from 1 to 3.

11. Water-soluble rylene dye according to any one of claims 1 to 10, wherein the linking group A is independently selected from 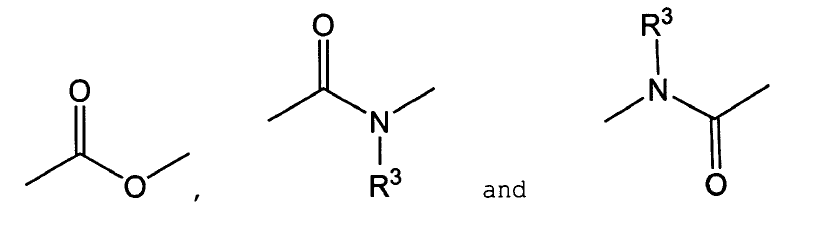 wherein R³ is independently selected from hydrogen, linear or branched-chain C₁-C₁₀ alkyl.

12. A rylene diimide compound of formula (I) 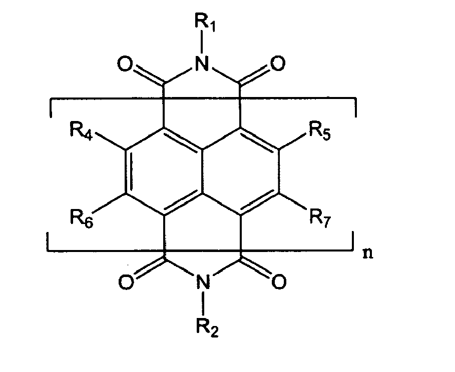 wherein R₁ and R₂ are independently an organic residue which is attached to the nitrogen atom via a carbon atom, n is an integer from 3 to 6,
wherein at least one of R₁ and R₂ contains a coupling group B which is selected from the group consisting of isothiocyanate, activated ester, carbonyl azide, sulfonyl chloride, aldehyde, alkyl halide, haloacetamide, maleimide, nitrilotriacetic acid, disulfide, thiosulfate, thiol and thioester,
and R₄, R₅, R₆ and R₇ are independently hydrogen or an organic residue, which carries at least one negatively or positively charged group in neutral medium or at least one hydrophilic uncharged group, wherein at least two of R₄, R₅, R₆ and R₇ are an organic residue, which carries at least one negatively or positively charged group in neutral medium or least one hydrophilic uncharged group.

13. A method for preparing a rylene diimide compound of formula (I) as defined in any one of claims 1 to 12, comprising the steps
(a) optionally reacting a rylene diimide compound of formula (IV) 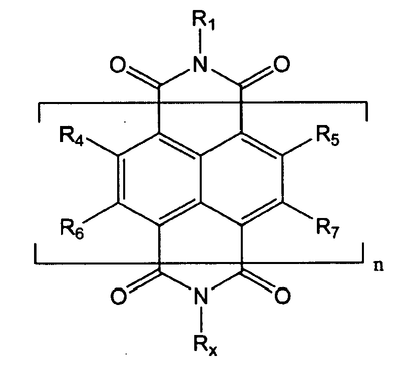 wherein R₁ and Rₓ are independently an organic residue which is attached to the nitrogen atom via a carbon atom and R₄, R₅, R₆ and R₇ are independently at each occurence hydrogen or an organic residue, to give a compound of formula (V), 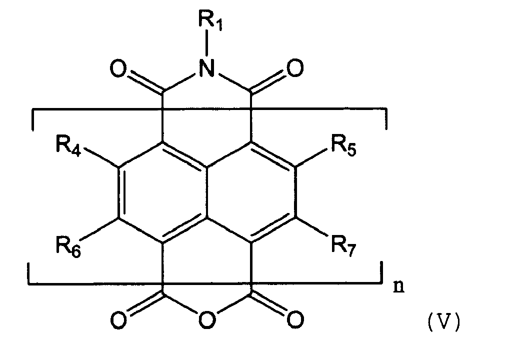
(b) reacting a compound of formula (V), to give a compound of formula (VI) 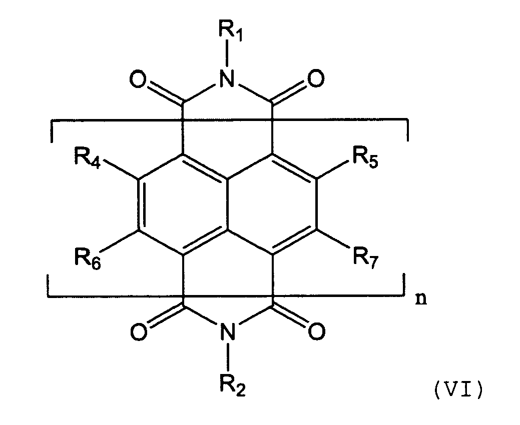 wherein R₂ is -(CH₂)_{q}COOH or -(CH₂)_{q}NHR⁸, wherein q is an integer from 1 to 20 and R⁸ is selected from hydrogen and linear or branched-chain C₁-C¹⁰ alkyl,
(c) reacting a compound of formula (VI), to give a rylene diimide compound of the following formula (I), wherein R₂ is -(CH₂)ₘ-A-(CH₂)ₒ-B, m is an integer from 1 to 20, o is an integer from 0 to 20,
A is 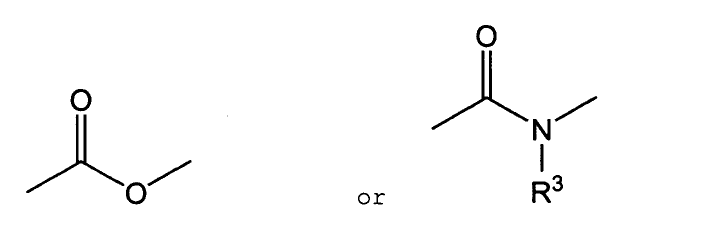 B is a coupling group selected from the group consisting of isothiocyanates, activated esters, carbonyl azides, sulfonyl chlorides, aldehydes, alkyl halides, haloacetamides, imides, disulfides, thiosulfates, thiols and thioesters, and
d) optionally further reacting the compound of formula (I) from step (b), wherein A is 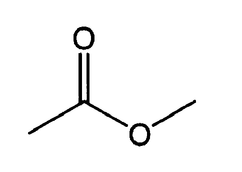 to give a compound of formula (I) wherein A is

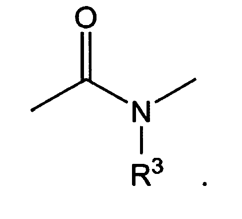

14. The method according to claim 13, comprising
(a) saponification of the Rₓ residue of formula (IV) under basic conditions and removal of the imide group of Rₓ, to give perylene monoimide monoanhydride compounds of formula (V);
(b) removal of the monoanhydride group of the compound of formula (V) by an imidization reaction with an 1,2-alkyl diamine or an 3-aminoalkanoic acid, respectively, to give monofunctionalized perylenes of formula (VI), wherein R₂ is -(CH₂)_{q}-COOH or -(CH₂)_{q}-NHR⁸, respectively,
(c1) reacting compound (VI) to give an intermediate product wherein at least two of R₄, R₅, R₆ and R₇ are an organic residue which carries at least one negatively or positively charged group in neutral medium;
(c2) reacting the product of step c1) with a compound providing a coupling group B as defined in claim 13 to give a compound of formula (I) as defined in claim 1 or claim 12.

15. The method according to claim 14, wherein step c1) comprises sulfonating the compound of formula (VI) under acidic conditions.

16. The method according to any one of claims 13 to 15,
wherein the coupling group B is an activated ester group, maleimide, thiol or thioester.

17. A method for preparing a water-soluble nitrilotriacetic acid-functionalized rylene dye of the formula (I), comprising
reacting a compound of formula (I) wherein the coupling group B is an activated ester group in solution with a compound providing a protected nitrilotriacetic acid group, and
subsequently removing the protective groups to give a compound of formula (I) wherein the coupling group B is an nitrilotriacetic acid group.

18. A method for preparing a water-soluble nitrilotriacetic acid-functionalized rylene dye of the formula (I), comprising coupling a carboxyl-functionalized rylene derivative with a nitrilotriacetic acid moity by amide bond formation in solid phase.

19. Conjugates, comprising a compound according to any one of claims 1-12 and a binding partner.

20. The conjugates according to claim 19, wherein the binding partner is a biomolecule.

21. Use of a rylene dye according to any one of claims 1-12 as fluorescent label for biomolecules.

22. Use of a rylene dye according to claim 4 as site-specific fluorescent label for proteins.

23. The use according to claim 22, wherein the coupling group of the rylene dye is a thioester for specific N-terminal labelling of proteins.

24. The use according to claim 22, wherein the coupling group of the rylene dye is a nitriotriacetic acid group for specific labelling of polyhistidin tagged proteins.

25. A method for purifying bioconjugates which contain the biomolecule and a dye as label, comprising the steps:
(a) providing a labelling solution comprising bioconjugates and non-reacted dye,
(b) adding a solid support to the labelling solution, wherein the non-reacted dye covalently couples to the solid support, and
(c) isolating the bioconjugates by removal of the solid support.

26. The method according to claim 25, wherein the dye is a rylene dye, in particular a perylene dye or terrylene dye.

27. The method according to claim 26, wherein the rylene dye is a rylene diimide dye according to any one of claims 1-12.

28. The method according to any one of claims 25-27, wherein the solid support is a resin carrying polyethylene glycole with at least one terminal amino group.
